# EUROPEAN PATENT SPECIFICATION

(11) **EP 2 854 848 B1**
(45) Date of publication and mention of the grant of the patent: **26.10.2016**
(21) Application number: 13724297.0
(22) Date of filing: 24.05.2013
(51) Int. Cl.: A61K 39/395

(54) **SECRETORY IMMUNOGLOBULIN DEFICIENCY TREATMENT AND PROPHYLAXIS**
BEHANDLUNG UND PROPHYLAXE VON SEKRETORISCHEM IMMUNOGLOBULINMANGEL
TRAITEMENT ET PROPHYLAXIE D'UNE DÉFICIENCE EN IMMUNOGLOBULINE SÉCRÉTOIRE

(30) Priority: 25.05.2012 EP 12169525
(43) Date of publication of application: 08.04.2015
(73) Proprietor: Himmler, Gottfried, 2301 Groß-Enzersdorf (AT)
(72) Inventor: Himmler, Gottfried, 2301 Groß-Enzersdorf (AT)
(74) Representative: Redl, Gerda
(86) International application number: PCT/EP2013/060724
(87) International publication number: WO 2013/174971

(56) References cited:
- WO-A1-02/076502
- WO-A1-2009/074539
- WO-A2-2007/057748
- DATABASE MEDLINE [Online] US NATIONAL LIBRARY OF MEDICINE (NLM), BETHESDA, MD, US; March 2012 (2012-03), LIMA MAYARA S R ET AL: "Influence of postpartum supplementation with vitamin A on the levels of immunoglobulin A in human colostrum.", XP009171099, Database accession no. NLM22406868 & LIMA MAYARA S R ET AL: "Influence of postpartum supplementation with vitamin A on the levels of immunoglobulin A in human colostrum.", JORNAL DE PEDIATRIA 2012 MAR-APR, vol. 88, no. 2, March 2012 (2012-03), pages 115-118, ISSN: 1678-4782
- BARRINGTON JULIAN W ET AL: "Immunoglobulin deficiency and recurrent postmenopausal endometritis", AMERICAN JOURNAL OF OBSTETRICS AND GYNECOLOGY, vol. 171, no. 5, 1994, pages 1389-1390, XP009171097, ISSN: 0002-9378
- CLARKE N M ET AL: "Effect of antimicrobial factors in human milk on rhinoviruses and milk-bourne cytomegalovirus in vitro", JOURNAL OF MEDICAL MICROBIOLOGY, SOCIETY FOR GENERAL MICROBIOLOGY, GB, vol. 49, 30 June 2000 (2000-06-30), pages 719-723, XP002558016, ISSN: 0022-2615
- Egbert Neike: "Kampf Durchfall und Pneumonie", , 5 July 2010 (2010-07-05), XP002702811, Retrieved from the Internet: URL:http://www.smul.sachsen.de/lfulg/downl oad/DruckNeike_Koellitsch_080416.pdf [retrieved on 2013-07-16]

## Description

### FIELD OF THE INVENTION

The invention refers to a preparation of secretory immunoglobulin (SIg) for use in the therapy or prophylaxis of mucosal immunoglobulin deficiency, and specific oral immunoglobulin preparations.

### BACKGROUND

The epithelial lining of mucous membranes covers an area of several hundred square metres in an adult, i.e. the airways, the gut, the conjunctiva covering the eyes, the urinary and genital tracts. It is the most frequent portal of entry for common infectious agents, allergens and carcinogens. The mucosa is protected by secretory immunoglobulins (SIg, secretory IgA and/or secretory IgM) amongst other factors. Secretory IgA (SIgA) consists of two Y-shaped IgA-antibody molecules connected tail-to tail covalently by a polypeptide called the J chain. Secretory IgM (SIgM) consists usually of five Y-shaped IgM-class-antibody molecules connected by the J chain. These polymeric antibodies are covalently linked to the "secretory piece" or "Secretory Component" (SC, which is the extracellular part of the poly-immunoglobulin receptor, pIgR) during secretion.

Secretory immunoglobulin plays a major role in the innate and adaptive immunoglobulin defence of epithelial surfaces and it is by far the most abundant immunoglobulin class in man.

IgA and IgM are present in healthy individuals in serum as well as at mucosal surfaces. In serum, monomeric IgA, polymeric IgM as well as dimeric IgA can be found whereas at mucosal surfaces and secretions, secretory IgA is predominant, secretory IgM being present in smaller amounts.

One of the most important molecules for protection against infection of humans and animals at mucosal sites (eyes, nose, mouth, lung, ears, trachea, esophagus, gastric tract, intestine, urogenital tract and colon) is secretory IgA which may act both via its four antigen binding sites as well as via the glycan-mediated binding of the Secretory Component.

Many studies demonstrate strong correlations between titers of specific SIgA antibodies in secretions and resistance to infection. BARRINGTON JULIAN W ET AL: "Immunoglobulin deficiency and recurrent postmenopausal endometritis",AMERICAN JOURNAL OF OBSTETRICS AND GYNECOLOGY, vol. 171, no. 5, 1994, pages 1389-1390 discloses that secretory immunoglobulin A is the principal immunoglobulin of mucosal surfaces and may be reduced in postmenopausal women because of estrogen deficiency. This can manifest as recurrent infections and may be a cause of undiagnosed postmenopausal bleeding. It is found that regular serum immunoglobulin therapy is appropriate for these patients.

Some studies demonstrate protection against systemic challenge with capsule forming bacterial pathogens.

Saliva and colostrum from normal subjects contain polyreactive SIgA and SIgM antibodies which recognize a variety of autoantigens and several bacterial antigens. It has been suggested that these are products of B-1 cells or at least cells that do not generate hypermutated antibodies, constituting part of the "natural antibody" repertoire encoded in the germline, and lacking memory capability and affinity maturation. They are considered to be the innate humoral immune system. Natural antibodies carry germline sequences that lack the variation in specificities that is brought about through variable-(diversity)-joining (V(D)J) region rearrangement, and they preferentially use D-proximal V heavy chain (VH) sequences. These antibodies may provide protection of the mucosal surfaces prior to the generation of specific antibodies from conventional B-2-cells after exposure to nominal antigens. Although they have low intrinsic affinity for antigens, the presence of 4 antigen-binding sites in SIgA and 10 antigen-binding sites in SIgM increases its functional activity.

In humans there are two unique IgA-subclasses (IgA1 and IgA2). Various allotypes of human IgA2 have been described (different combinations of constant region domains of the alpha-heavy chains). The predominant molecular form of circulating (plasma) IgA is monomeric, in contrast to the dimeric (polymeric) pIgA produced in epithelium which is mostly transported into the secretions as SIgA.

Human IgA1 and IgA2 (including allotypes) appear to have few distinct biologically properties, e.g. their different susceptibility to bacterial proteases. IgA1 and IgA2 also differ in the distribution of antibody specificities.

Immunization of adults with protein antigens elicits mainly IgA1 and immunization with polysaccharides provokes mainly an IgA2 antibody response. Of the immunoglobulin isotypes that reach mucosal surfaces, SIgA is one of the most stable and this stability has been largely ascribed to the Secretory Component which masks potential cleavage sites within the Fc-portion.

The specificity of SIgA antibodies for surface structures of microbial surfaces to inhibit adherence to pharyngeal, intestinal, genitourinary tract and gingival epithelia was demonstrated. In addition to a specific, antibody-mediated inhibition of adherence, human IgA, and SIgA in particular, bind to many bacterial species and antigens by means of their carbohydrate chains. A notable example of this is seen in the case of IgA2, which can agglutinate *E*. *coli* by a mechanism involving the type I (mannose dependent) pili and type I pilus-dependent adherence of *E. coli* to epithelial cells.

IgM in external secretions is also associated with the Secretory Component (secretory IgM, SIgM) resulting from its transport into secretions by the pIgR. The concentration of SIgM is lower than that of SIgA either because of the lower proportion of IgM-producing cells in mucosal tissues or because IgM may be less well transported than pIgA due to a molecular weight restriction in pIgR-dependent transport.

Natural antibodies, by definition, are produced in the apparent absence of antigenic stimulation. They are produced by a specific subset of B-cells and do not extensively affinity mature. Natural antibodies of the classes IgA, IgM and IgG have been described. These antibodies are encoded usually by germline genes with few, if any, mutations and have in many cases broad reactivity against PAMPs (pathogen-associated molecular pattern), tumor antigens and a number of autoantigens. Because of their low affinity and germ-line configuration, such polyreactive antibodies do not appear to be true autoantibodies and certainly do not fit into the same category as antigen-specific, somatically-mutated, high affinity pathological autoantibodies.

Natural antibodies have been proposed for certain therapeutic uses, e.g. cancer therapy or in infectious diseases.

Many of the polyreactive antibodies have a germ-line or near germ-line sequence and are primarily IgM, but some are also IgG and IgA.

Contrary to the classic "lock and key" rigid structure hypothesis of antigen-antibody interaction, the antigen binding pocket of polyreactive or multispecific antibodies, perhaps because of their germ-line configuration, are believed to be more flexible and therefore can accommodate different antigenic configurations.

Although some reports have suggested that SIgA and SIgM are polyreactive in nature, other findings point to a restricted specificity that may be cross-reactive.

The Secretory Component (SC) has been described to occur in various body secretions such as saliva, tears, mucus and milk. It can be found either as part of secretory immunoglobulins (SIgA and SIgM) as well as free Secretory Component (fSC).

Human Secretory Component (hSC) is derived from the polymeric immunoglobulin receptor by cleavage of the extracellular part of the receptor molecule in the process of transcytosis. It has an apparent molecular weight of about 80 kDa and consists of the first about 585 amino acids of the pIgR (polymeric Ig-Receptor) arranged in five V-type immunoglobulin domains. It has 7 potential N-glycosylation sites. This strong glycosylation contributes to the large apparent molecular weight. The composition of these glycans includes bi- and triantennarry structures, Lewis type or blood group related structures as well as galactose and sialic acids. These glycans constitute binding epitopes for bacterial, viral, fungal and protozoan structures such as adhesins and toxins.

Proposed functions of the Secretory Component are the protection of polymeric immunglobulin from proteolytic degradation, binding to receptors such as DC-SIGN and binding to pathogen related structures and toxins such as Helicobacter pylori, enteropathogenic *E.coli, Clostridium difficile* toxin A and *Streptococcus pneumoniae* cholin binding protein A. Glycans on SC have proven to participate in innate protection against mucosal pathogens (Perrier et al. THE JOURNAL OF BIOLOGICAL CHEMISTRY Vol. 281 (20), pp. 14280-14287, 2006). hSC was identified as a microbial scavenger contributing to the antipathogenic arsenal that protects the body epithelial surface.

SC purified from human milk was found to competitively inhibit *Clostridium difficile* toxin A binding to receptors (Dallas et al. J. Med. Microbiol. 47: 879-888 (1998)). Removing carbohydrates from SIgA and SC by enzymatic digestion showed that *Clostridium difficile* toxin A binds much less to deglycosylated SC than to glycosylated SC.

There are a number of physiological and disease states which influence the secretory or mucosal immune defence. A frequent reason for an impaired immune defence is an immunodeficiency (primary or aquired). Some of those immune deficiencies also influence the mucosal immune defence.

Common variable immunodeficiency (CVID) (also known as Acquired hypogammaglobulinemia; ICD10 D83) is a group of approximately 150 primary immunodeficiencies (PIDs), which have a common set of symptoms (including hypogammaglobulinemia) but which have different underlying causes.

Common variable immunodeficiency is the most commonly encountered primary immunodeficiency.

Diagnosis of CVID is usually made by demonstrating low levels of immunoglobulins in the serum.

Selective Immunoglobulin A deficiency (ICD-10 D80.2) is defined as decreased or absent level of serum IgA in the presence of normal serum levels of IgG and IgM in a patient older than 4 years of age, in whom other causes of hypogammaglobulinemia have been excluded. Serum IgA level of less than 7 mg/dL (0.07 g/L) is considered as selective IgA deficiency. Serum IgA levels higher than 7 mg/dL but two standard deviations below that normal for age, the condition may be referred to as partial IgA deficiency, which is quite common. The threshold of 4 years of age is used to avoid premature diagnosis of IgA deficiency which may be transient in younger children due to delayed development of the IgA system.

Although individuals with selective IgA deficiency do not produce IgA, they do produce all the other immunoglobulin classes. In addition, the function of their T-lymphocytes, phagocytic cells and complement system are usually normal. The causes of selective IgA deficiency are unknown. It is likely that there are a variety of causes for selective IgA deficiency and the cause may vary from individual to individual.

Selective IgA deficiency is considered as the most common primary immunodeficiency. The worldwide incidence has been reported to depend on the ethnic background: 1:143 in the Arabian peninsula, 1:163 in Spain, 1:252 in Nigeria, 1:875 in England and 1:965 in Brazil. In general, IgA deficiency is more common in Caucasians. In the United States, the frequency is estimated to be from 1:223 to 1:1,000 in community studies and from 1:333 to 1:3,000 among healthy blood donors.

Approximately 80% of IgA-deficient individuals are asymptomatic. The variability in clinical presentation, familial patterns, genetic markers, abnormalities of immunocytes and the concentrations of other serum immunoglobulins suggests that IgA deficiency may be caused by several different immunological defects. IgA-deficiency has been linked to the MHC (HLA B8.1 allele) in approximately 20% of patients.

The most common immunologic abnormality in most IgA-deficiencies is the failure of IgA surface expressing cells to differentiate into IgA producing plasma cells. However, some patients have been reported to have low expression of membrane and secreted isoforms of C-alpha and impaired class switching to IgA. In rare cases can IgA-deficiency be attributed to a specific genetic deletion of a portion of the CH-alpha gene. Other underlying immunologic mechanisms may be IgA-specific suppressor T cells, inadequate helper T cell function or B cell impairment.

A dysregulation of cytokine responses may contribute to IgA deficiency. Serum levels of TGF-beta in IgA-deficient patients have been shown to be below normal levels. Incubation of B-cells from some IgA-deficient patients with exogenous IL-10 can correct their IgA production defects.

IgA deficiency may be induced by drugs such as captopril, sulfasalazine, cyclosporine, cyclophosphamide, hydantion, fenclofenac, gold salts, penicillamine, sodium valporate.

IgA-deficiency may further be induced by stress (e.g. extreme physical exercise and sports or infections) and by malnutrition (e.g. Vitamin A deficiency, Vitamin D deficiency, lack of iron, zinc, etc.).

It is reported that IgA deficiency may occur after surgery such as tonsillectomy, appendectomy or certain gastric bypass (Roux-en-Y).

Secretory IgA deficiency is understood as a deficiency in secretory IgA.

It may be that the serum IgA level is normal but the production of dimeric IgA is impaired, e.g. by low or absent expression of J-chain. Another form of secretory IgA deficiency may be caused by low or impaired expression of the pIgR which consequently reduces or abolishes transport of polymeric immunoglobulin to the mucosal surfaces. It is is common understanding that routine screening for Secretory Component deficiency and thus secretory IgA deficiency is not valuable because of the rarity of the defect (J Allergy Clin Immunol. 1991, volume 88 pages 356-360).

Secretory IgA deficiency may be caused by local disturbance of SIgA production in the mucosa-associated lymphoid tissue (MALT) e.g. by stress, malnutrition, injury or surgery.

Secretory IgA deficiency is not necessarily correlated to selective IgA deficiency.

IgA deficiencies may be transient (lasting several days to several years) and/or restricted to local compartments such as gastric tract, urogenital tract, mouth, nose, lung or eyes.

Several studies have been performed on subjects with no overt immunodeficiency in order to see if low levels or lack of salivary IgA shows any relationship to contraction of allergy or infectious disease, mainly in the upper airways. Infants born to atopic parents have been shown a significantly increased prevalence of salivary IgA deficiency, this deficiency presumably mainly being transient. Such a temporary IgA defect has been shown to correlate weakly to later development of allergy. The correlation may even be stronger if compensation of lacking SIgA by SIgM is taken into account.

It has also been reported that total salivary IgA tends to be reduced in infection-prone children with no overt immunodeficiency. Salivary IgA may, moreover, be decreased in children with recurrent tonsillitis or adenoid hyperplasia, and also in asthmatic children when wheezing is precipitated mainly by recurrent respiratory tract infections. A significant relationship of transient salivary IgA deficiency with bronchial hyperreactivity has been reported.

It has also been suggested that repeated antibiotic courses may lead to persistently low salivary IgA levels. AIDS patients or HIV-positive individuals are also reported to have secretory IgA deficiency.

It is not understood why some individuals with IgA deficiency have almost no illness while others are very sick. One of the reasons may be a compensatory IgM production. Another explanation could be the diagnosis of IgA deficiency via serum IgA although secretory IgA in various compartments of the body may be independently regulated and disturbed. Also, it is not known precisely what percent of individuals with IgA deficiency will eventually develop complications; estimates range from 25% to 50% over 20 years of observation.

Selective immunoglobulin M deficiency (ICD-10 D80.4) is a rare form of dysgammaglobulinemia characterized by an isolated low level of serum immunoglobulin M (IgM). Reported IgM concentrations in selective IgM deficiency vary from 40 mg/dL to undetectable levels (normal reference range is 45-150 mg IgM/dL serum in adults and 5-15 mg IgM/dL in children), while the levels of other immunoglobulin classes are within reference ranges.

Selective IgM deficiency may occur as a primary or secondary condition. Secondary selective IgM deficiency is much more common than primary selective IgM deficiency and may be seen in association with malignancy, autoimmune disease, gastrointestinal disease, and immunosuppressive treatment.

Selective IgM deficiency is rare, with an incidence of less than 0.03% in the general population and 1% in hospitalized patients and is usually discovered during the investigation of other conditions, such as autoimmune disease or malignancy. Patients with selective IgM deficiency are susceptible to recurrent sepsis and overwhelming infection with encapsulated bacteria (e.g. Streptococcus pneumoniae, Neisseria meningitidis, Haemophilus influenzae). They may also have autoimmune disease including glomerulonephritis and osteomyelitis as well as malignancies, chronic dermatitis, diarrhea and upper respiratory infections.

Some patients with selective IgA or IgM deficiency have a tendency to develop recurrent sinopulmonary infections, gastrointestinal infections and disorders, allergies, autoimmune conditions, and malignancies. Some people with low serum IgA and/or IgM have a clinical course very similar to people with common variable immunodeficiency.

A common problem in IgA deficiency and/or IgM deficiency is susceptibility to infections. This is seen in about half of the patients with IgA and/or IgM deficiency who come to medical attention. Recurrent ear infections, sinusitis, bronchitis and pneumonia are the most common infections seen in patients with selective IgA and/or IgM deficiency.

These infections are mostly due to bacteria, e.g. Haemophilus influenzae and Streptococcus pneumoniae. Some patients may develop end organ damage such as bronchiectasis secondary to recurring or chronic infections. Patients with associated antibody deficiency such as IgG2 subclass deficiency have a higher chance of having more severe infections and complications.

Some patients also have gastrointestinal infections and chronic diarrhea.

Giardiasis, malabsorption, lactose intolerance, celiac disease, ulcerative colitis, nodular lymphoid hyperplasia, and malign proliferation are among the associated diseases.

Another major problem in IgA and/or IgM deficiency is the occurrence of autoimmune diseases. These are found in about 25% to 33% of patients who seek medical help. In autoimmune diseases, individuals produce antibodies or T-lymphocytes which react with their own tissues with resulting inflammation and damage. Autoantibodies, such as antibodies against sulfatide, Jo-1, SM, cardiolipin, phosphatidylserine and collagen have be detected in IgA-deficient patients even without overt clinical disease.

Some of the more frequent autoimmune diseases associated with IgA and/or IgM deficiency are idiopathic thrombocytopenic purpura, hemolytic anemia, juvenile rheumatoid arthritis, thyroiditis and systemic lupus erythematosus.

These autoimmune diseases may cause sore and swollen joints of the hands or knees, a rash on the face, anemia or thrombocytopenia.

Other kinds of autoimmune disease may affect the endocrine system and/or the gastrointestinal system. For instance, patients with IgA deficiency have a higher chance of developing celiac disease. Patients with IgA deficiency are not expected to develop IgA isotype antibodies against gliadin, tissue transglutaminase, or endomysium; however, they may have IgG isotype antibodies against those antigens. Symptoms associated with food allergies are diarrhea or abdominal cramping.

Inflammatory bowel diseases, mostly ulcerative colitis, have also been reported in association with selective IgA and/or IgM deficiency.

Allergies may also be more common among individuals with selective IgA and/or selective IgM deficiency than among the general population. These occur in about 10-15% of these patients. The types of allergies vary. Asthma is one of the common allergic diseases that occur with selective IgA deficiency. There may be an also an increased incidence of allergic rhinitis (hay fever) or eczema in selective IgA deficiency.

Treatments of the problems associated with selective IgA and/or IgM deficiency are usually directed towards the particular problem. For example, patients with chronic or recurrent infections need appropriate antibiotics. Certain patients who have chronic sinusitis or chronic bronchitis may need to stay on long term preventive antibiotic therapy.

There are a variety of therapies for the treatment of autoimmune diseases. Antiinflammatory drugs, such as aspirin or ibuprofen, are used in diseases that cause joint inflammation. Steroids are helpful in a variety of autoimmune diseases. If autoimmune disease results in an abnormality of the endocrine system, replacement therapy with hormones may be necessary.

Treatment of the allergies associated with IgA and/or IgM deficiency is similar to treatment of allergies in general.

Treatment of various forms of immunoglobulin deficiency such as CVID is done by administering immunoglobulin from plasma. It has been shown that IgG replacement therapy can safely be given to patients with selective IgA deficiency and the treatment seemed to be effective as the frequency of bacterial respiratory tract infections was reduced (The Lancet, 1997, volume 350, page 865).

Most immunoglobulin preparations from human plasma contain mainly IgG. Although there are also preparations containing IgM and IgA they do not contain the secretory immunoglobulins SIgA and SIgM.

Milk does contain - amongst other immunoglobulins - SIgA and SIgM. Immunglobulin preparations from milk have been used for treatment of various diseases (for a review on hyperimmune milk antibodies see e.g., Adv Exp Med Biol; 2008; volume 606, pages 321-343).

Merhav et al. (Transplant International (1995) 8(4) 327-329) describe treatment of serum IgA deficiency in liver transplants recipients with human milk.

Harabuchi et al. (Journal of Pediatrics (1994) 124(2): 193-198) describe passive immunization of babies using human milk comprising specific anti-Haemophilus influenza antibodies.

WO2009139624A1 discloses a process for producing compositions that are rich in secretory IgA by fractionating non-human milk. Such compositions may be used in particular for treating and/or preventing infections and/or inflammation of the mucosal surfaces, e.g. the gastro-intestinal tract, urogenital tract, respiratory tract, nasal cavity or oral cavity, treating and/or preventing obesity and related diseases, or treating and/or preventing food allergies in subjects in need of such treatment.

Exogenous IgA has been topically applied to the nose in both animals and humans for the purpose of preventing and treating disease. In humans, nasal administration of a preparation of approximately 70% serum IgA and 30% serum IgG resulted in decreased frequency of upper respiratory tract infections in high risk groups, such as elite skiers and children.

It was proposed that oral supplementation of IgA would be beneficial for those neonates who are at particular risk for gut-origin sepsis (Annals of the New York Academy of Sciences, volume 778, page 405-407, 1996).

Oral immunoglobulin containing IgA and IgG from plasma has been tested for use for preventing necrotizing enterocolitis in preterm and low birth-weight neonates (Foster and Cole, Oral immunoglobulin for preventing necrotizing enterocolitis in preterm and low birth weight neonates (Cochrane Database of Systematic Reviews 2004, Issue 1. Art. No.: CD001816).

WO2002076502 describes the production and use of secretory IgA by combining IgA from pooled human plasma with recombinant J-chain and recombinant secretory component.

Carbonare et al. (in Pediatr Allergy Immunol, 2005, volume 16, pages 574-581) describe the preparation of SIgA suitable for oral use (from human colostrum and milk), which may be administered to immunodeficient patients with gastrointestinal manifestations.

EP479597B1 describes a process for producing secretory immunoglobulin-A preparations effective in the cases for which supplementary local immunotherapy on mucosa can be applied: Primary immunodeficiency syndrome and difficult to treat diarrhoea and aphthous stomatitis accompanied by secondary immunodeficiency (immunodeficiency by infection, nutritional disorders, drugs, etc.). It was suggested that the secretory IgA is effective in treating recurrent upper respiratory inflammation or otitis media and in treatment after operation of biliary obstruction.

WO2009074539 describes IgA enriched milk suitable for use in the prevention or treatment of otitis media comprising IgA derived from mature bovine milk and having specificity for at least one of Streptococcus pneumoniae, Haemophilus influenzae and Moraxella catarrhalis. A specific infant formula is described to also contain all vitamins and minerals understood to be essential in the daily diet and in nutritionally significant amounts, including Vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine.

There is a need for treatment of transient, aquired and chronic immunodeficiency in order to avoid development of recurrent infections, allergies and autoimmune diseases.

### SUMMARY OF THE INVENTION

It is the objective of the present invention to provide improved therapy of subjects at risk of immunodeficiency disease conditions.

The objective has been solved by the subject matter of the claims.

According to the invention there is provided a secretory immunoglobulin (Slg) preparation comprising SIgA and/or SIgM, herein also called an SIgA and/or SIgM preparation, and a Vitamin A supplement, for use in the therapy or prophylaxis of mucosal immunoglobulin deficiency, such as secretory immunoglobulin deficiency, specifically a preparation comprising an effective amount of SIgA and/or SIgM and Vitamin A.

Specifically, the preparation comprises polyclonal or polyreactive SIgA and/or SIgM, preferably derived from milk of animals selected from the group consisting of human, cow, pig, goat, sheep, buffalo, horse, donkey and camel.

According to specific embodiments, the Vitamin A comprised in the Slg preparation according to the invention is a molecule with Vitamin A activity, e.g. selected from the group consisting of retinal, retinol, retinoids, retinoic acids, such as all trans-retinoic acid or 13-cis-retinoic acid, retinylpalmitate, 3-dehydroretinol, 3-dehydroretinal, beta-carotene, alpha-carotene, gamma-carotene, beta-cryptoxanthineretinol, and functionally active derivatives thereof.

It is preferred that the preparation according to the invention further comprises at least one additive or supplement selected from the group consisting of Vitamin B2, Vitamin B6, Vitamin B12, Vitamin C, Vitamin D, Vitamin E, zinc and selenium.

Specifically, the preparation is provided in a formulation for mucosal use, preferably oral, nasal, vaginal, intragastric or rectal use.

According to a specific aspect, a subject is treated that is at risk of infections, allergies and autoimmune diseases. Specifically, the subject is suffering from IgA and/or IgM deficiency, including selective IgA and/or IgM deficiency, SIgA deficiency and/or SIgM deficiency, and specifically a combined secretory IgA/IgM deficiency.

According to a further specific aspect, the subject is treated with an oral preparation, e.g. to provide a single-dose of 10 mg to 10 g SIgA and/or SIgM, e.g. a preparation wherein either of the SIgA or the SIgM is contained in an amount of 10 mg to 10 g per administration unit, e.g. as the predominant immunoglobulin, or a combination of the SIgA and SIgM in the amount of 10 mg to 10 g in total.

Preferably, the preparation according to the invention is provided as a liquid, syrup, lozenge, tablet, chewing gum, spray, powder, instant powder, granules, capsules, cream, gel, drops, suspension, emulsion or food product, for example, including specific excipients or auxiliary means for providing the respective formulation.

Specifically the preparation according to the invention comprises at least 40% secretory immunoglobulins SIg, in particular SIgA and/or SIgM, of the total immunoglobulin content, preferably at least 50%, at least 60%, at least 70%, at least 80% or at least 90%. Thus, it is preferred that the IgG content in the preparation is less than 60%, preferably less than 50%, less than 40%, less than 30%, less than 20% or less than 10% of the total immunoglobulin content.

The preparation according to the invention preferably is enriched in SIg or comprises the SIg in the concentrated form, e.g. at least 18 mg SIgA per gram and/or 10 mg SIgM per gram. It is further preferred that the preparation according to the invention contains a reduced amount of other milk components relative to the immunoglobulins, e.g. less than 1 mg lactalbumin per gram. It is also preferred to employ a preparation comprising at least 1 mg SIgA and/or SIgM per gram, wherein
other milk components, such as lactalbumin are reduced or depleted, e.g. to a level of less than 1 mg lactalbumin per gram.

According to a specific aspect, there is provided a method to determine the SIgA and/or SIgM content in a mucosal sample of said subject, which is compared to a reference value, for diagnosing SIgA and/or SIgM deficiency, in particular mucosal IgA and/or mucosal IgM deficiency. Accordingly, the invention provides for a preparation according to the invention and its specific use, wherein the mucosal immunoglobulin deficiency, such as a SIgA and/or SIgM deficiency, is determined in a mucosal sample of said subject in comparison to a reference.

Specifically, the mucosal sample is derived from body fluids selected from the group consisting of saliva, gastric juice, tears, middle ear fluid, faeces, sweat, urine, gut lavage, middle ear fluid, bronchial lavage and nasal secretions.

According to a specific aspect, there is provided a method for treating a subject at risk of developing infections, allergies and autoimmune diseases, which method comprises the following steps:
- obtaining a sample of mucosal body secretions,
- determining the content of SIgA and/or SIgM in said sample in comparison to a reference, diagnosing a potential mucosal immunoglobulin deficiency, and
- treating said subject with a preparation according to the invention to treat said mucosal immunoglobulin deficiency, in particular either of an SIgA or SIgM, or both, an SIgA and SIgM deficiency.

Further provided herein is an oral immunoglobulin preparation comprising
- a single-dose of 10 mg to 10 g SIgA and/or SIgM, and
- a single-dose of 100 to 5000 micrograms retinol activity equivalents (RAE) Vitamin A, preferably retinylpalmitate,
and optionally further components selected from the group consisting of vitamins and minerals.

Specifically there is provided a preparation comprising 400-1200 mg Slg, e.g. 400-600 mg SIgA and/or SIgM, preferably about 500 mg SIg in total, and 700-900 microgram retinylpalmitate, preferably about 800 microgram.

### FIGURES:

Figure 1 shows the amino acid sequence of the heavy chain of the polyreactive antibody 2E4 IgA1 (2E4 Calpha1, SEQ ID NO: 3)
Figure 2 shows the amino acid sequence of the heavy chain of the polyreactive antibody 2E4 IgA2 (2E4 Calpha2, SEQ ID NO: 4)
Figure 3 shows the amino acid sequence of the heavy chain of the polyreactive antibody 2E4 IgG (2E4 Cgamma1, SEQ ID NO: 5)
Figure 4 shows the amino acid sequence of the kappa light chain of the polyreactive antibody 2E4 (2E4 kappa, SEQ ID NO: 6)

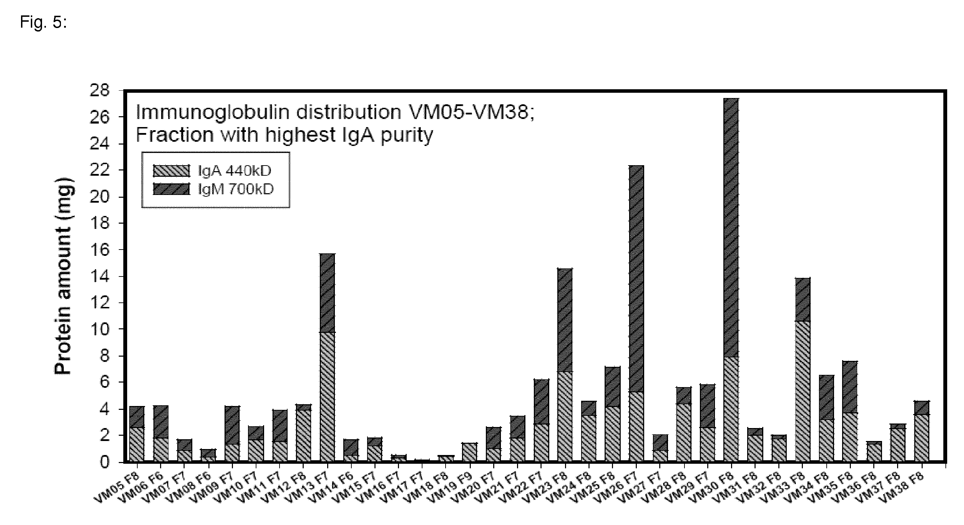

Figure 5: Quantitative immunoglobulin class distribution in secretory immunoglobulin preparations from goat milk.

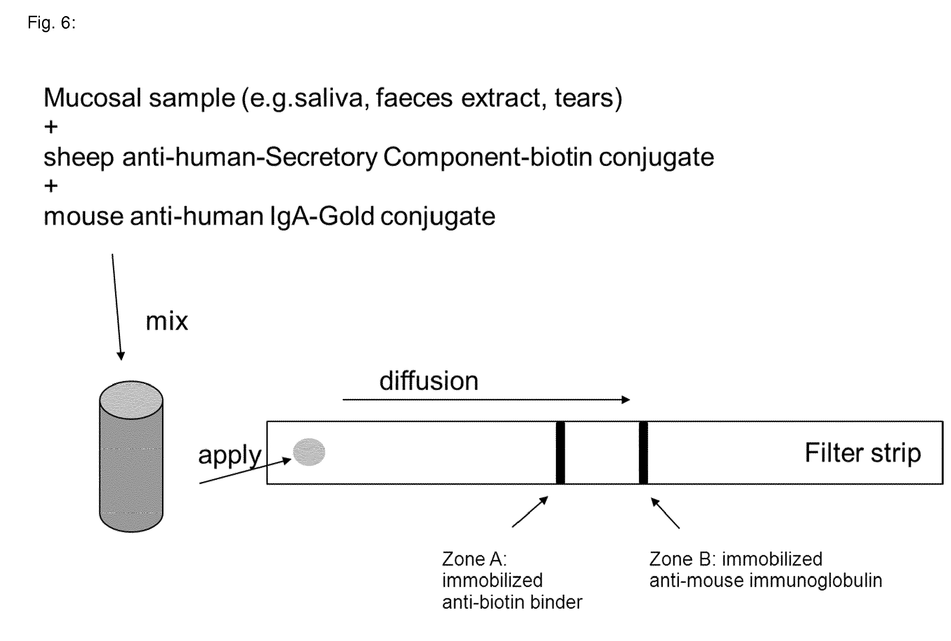

Figure 6: Rapid assay for determining SIgA.

### DETAILED DESCRIPTION OF THE INVENTION

Specific terms as used throughout the specification have the following meaning.

The term "eukaryotic host" shall mean any eukaryotic cell, tissue or organism, which may be cultivated to express a protein. Specifically, the eukaryotic host is a eukaryotic host cell line. It is well understood that the term does not include human beings. Preferred hosts are eukaryotic hosts, in particular to express the IgA, IgM and/or other components of the SIg, such as the J-chain and the Secretory Component.

The term "expression system" or "production system" as used herein shall refer to organisms like cell cultures or higher eukaryotic organisms, like selected lactating animals capable to produce proteins and immune complexes of the desired quality and quantity. Preferred systems employ expression vectors for use in a eukaryotic host.

"Expression vectors" or "vectors" as used herein are defined as DNA sequences that are required for the transcription of cloned recombinant nucleotide sequences, i.e. of recombinant genes and the translation of their mRNA in a suitable host organism. Such expression vectors may comprise an origin for autonomous replication in the host cells, selectable markers (e.g. an essential amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together.

The term "food" or "food product" shall comprise any compound, preparation, mixture, or composition suitable for, or intended for intake by an animal. This includes any compound that is a nutritional, nutraceutical or food supplement, dietary food, complete or incomplete balanced diet or supplement or medical food which is understood as nutritional or functional supplement to a food product, possibly used as a diet. Typically, functional food products aid in the prevention or prophylaxis and/or treatment of disease conditions associated with pathogens, including toxins or the treatment of physiological imbalances of the body. The term shall also comprise feed or feed products, possibly used as a diet for feeding non-human animals. Food may be of organic or synthetic sources, formulated in natural or natural-like compositions including dairy products or synthetic compositions based on artificial mixtures of substances, which have been suitably purified before mixing. The food product according to the invention typically is provided in food grade quality. The grade quality is the quality characteristics of food that is acceptable to animals. This includes external factors as appearance (size, shape, colour, gloss, and consistency), texture and flavour. Quality standards also provide for an acceptable maximum amount of contaminating substances. Besides ingredient quality, there are also sanitation requirements to inactivate or deplete pathogens. It is important to ensure that the food processing environment is as clean as possible in order to produce the safest possible food for the consumer.

The term "host cell" or "host cell line" refers to a microorganism or a cell line, used for expressing a recombinant gene to produce the recombinant proteins as used according to the invention. Preferred host cells are selected from the group consisting of mammalian, avian, insect or plant cells, yeasts, filamentous fungi or bacteria. For producing immunoglobulins or SIg as used according to the invention, host cells capable of producing glycoproteins such as the Secretory Component with Lewis-type N-glycosylation (i.e. peripheral or antennary fucosylation) are preferably used. A host cell clone of cultivated host cells that have proliferated is commonly understood to be a host cell line. A production host cell line is commonly understood to be a cell line ready-to-use for cultivation in a bioreactor to obtain the product in an industrial scale.

The term "isolated" or "purified" with respect to proteins as used herein, such as to specify the immunoglobulins or Secretory Component (SC) as used according to the invention, refers to a protein which is obtained from a complex mixture of an animal's body fluid or secretions, thus, of natural origin, or of cell cultures, like a cell culture supernatant or a tissue or cell extract. Those proteins are typically at least 50% pure, preferably at least 60% pure, more preferably at least 70% pure, even more preferably at least 80% pure, most preferably at least 90% pure, and even most preferably at least 95% pure, as determined by SDS-PAGE.

The term "polymeric immunoglobulin" as used herein shall refer to an association of at least 2, 3, 4, 5 or even a higher number up to 10 immunoglobulin molecules, in particular Y-shaped immunoglobulin molecules. The polymeric immunoglobulin is thus, considered an at least dimeric immunoglobulin, e.g. dimeric IgA, trimeric, quatromeric, pentameric, such as sIgM, hexameric immunoglobulin or even higher polymers or aggregates. Polymeric immunoglobulins may comprise the immunoglobulin molecules associated with each other by covalent bonding, or other interactions, like electrostatic, hydrophobic, ionic interactions or affinity binding with or without J-chain.

The term "polyreactive immunoglobulin" as used herein shall refer to an immunoglobulin with at least two specificities, meaning that it recognises at least two different epitopes, also known as cross-reactivity. Typically, polyreactive immunoglobulins of an innate immune system would have at least 3, 4, 5 or more relevant (e.g. with regard to physiologically relevant or pharmacologically active) specificities to bind epitopes and antigens, most commonly with low or medium affinities. Such multispecific antibodies may be able to bind at least two different epitopes simultaneously. Specifically included in this term are antibodies of germline origin.

The term "recombinant" as used herein refers to proteins (including polypeptides) produced by genetic engineering or gene recombination techniques employing a recombinant expression system, like host organisms, such as prokaryotes or eukaryotes, including transgenic organisms such as transgenic plants or transgenic animals, in a contained reactor system such as microbial fermentation or cell culture, e.g. employing a production host cell line or strain like a yeast, fungi, bacteria or archae, a cell line, from mammalian cells, insect cells, plant cells or respective tissues.

The term "Secretory Component" or "SC" as used herein shall refer to a secretory component that may be secreted by a mammary gland of an animal, including humans, or variants thereof, including functional variants, which SC is e.g. a glycoprotein separate from an immunoglobulin or in complex with an immunoglobulin, e.g. to form a secretory immune complex, e.g. mediated by the J-chain (or variants thereof) or other structures of immunoglobulins that bind specifically to the poly-immunglobulin receptor (plgR). An SC may be obtained from natural sources, such a colostrum or milk, or else produced synthetically or by recombinant expression techniques.

The term "secretory immunoglobulin" or "Slg" as used herein shall refer to an immunoglobulin that may be secreted by a mammary gland of an animal including humans, e.g. mediated by the pIgR or variants thereof, including functional variants, herein called functionally active variants. A secretory immunoglobulin may be obtained from natural sources, such a colostrum or milk, in particular as an SIgA and/or SIgM, or else produced synthetically or by recombinant expression techniques. The secretory immunoglobulin as used herein specifically is a non-denatured protein complex of polymeric immunoglobulin and secretory component able to bind to epitopes of antigens and receptors both via the variable regions of the immunoglobulin and the secretory component.

The term "variant" or "functionally active variant" of a protein like the Secretory Component or an immunoglobulin, as used herein means a sequence resulting from modification of the parent sequence by insertion, deletion or substitution of one or more amino acids or nucleotides within the sequence or at either or both of the distal ends of the sequence, and which modification does not affect (in particular impair) the activity of this sequence. In a preferred embodiment the variant is a functionally active variant, which a) is a biologically active fragment of the amino acid or the nucleotide sequence, the functionally active fragment comprising at least 50% of the sequence of the amino acid or the nucleotide sequence, preferably at least 60%, preferably at least 70%, more preferably at least 80%, still more preferably at least 90%, even more preferably at least 95% and most preferably at least 97%, 98% or 99%; b) is derived from the amino acid or the nucleotide sequence by at least one amino acid substitution, addition and/or deletion, wherein the functionally active variant has a sequence identity to the amino acid or the nucleotide sequence or to the functionally active fragment as defined in a) of at least 50%, preferably at least 60%, preferably at least 70%, preferably at least 80%, still more preferably at least 90%, even more preferably at least 95% and most preferably at least 97%, 98% or 99%; and/or c) consists of the amino acid or the nucleotide sequence and additionally at least one amino acid or nucleotide heterologous to the amino acid or the nucleotide sequence, preferably wherein the functionally active variant is derived from or identical to any of the naturally occurring variants of any of the sequences found in various gene and protein databases.

Any non-mutated (germline) or minimally mutated VhDJ combination and any germline Vkappa and Vlambda may be used to form the V-region of a polyspecific or cross reactive SIg molecule of the invention.

SC sequences specifically used in a preparation according to the invention are described in the prior art as sequences of complete polyimmunoglobulin receptors (pIgR) or as extracellular part of these sequences.

"Percent (%) amino acid sequence identity" with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithm as needed to achieve maximal alignment over the full length of the sequences being compared.

The functionally active variant may be obtained by sequence alterations in the amino acid or the nucleotide sequence, wherein the sequence alterations retain a function of the unaltered amino acid or the nucleotide sequence, when used in combination of the invention. Such sequence alterations can include, but are not limited to, (conservative) substitutions, additions, deletions, mutations and insertions.

In a specific embodiment of the invention, the polypeptide or the nucleotide sequence as defined above may be modified by a variety of chemical techniques to produce derivatives having essentially the same activity (as defined above for fragments and variants) as the modified polypeptide or the nucleotide sequence, and optionally having other desirable properties, like reactivity, N-glycosylation sites and stability (*in vivo* or *in vitro* stability). Desirable properties are, for example, the increase in thermostability and/or gastrointestinal stability, as measured by the pH stability and/or protease (e.g. pancreatic) stability of the protein. The glycosylation pattern of the immunoglobulin can affect numerous aspects of the therapeutic efficacy such as solubility, resistance to proteolytic attack and thermal inactivation, immunogenicity, half-life, bioactivity, receptor binding and stability.

Variants of the SIg as used according to the invention may have altered amino acid sequences to introduce additional glycosylation sites, in particular on the SC component of the SIg complex. A preferred embodiment of the invention is the addition of N-glycosylation sites, in particular Lewis-type glycosylation or other non-core fucosylation. This can be achieved by genetic engineering techniques as well as chemical and enzymatic means. Introduction of the sequence motif Asn-Xaa-Thr-Xaa (Seq. ID No. 1) or Asn-Xaa-Ser-Xaa (Seq. ID. No. 2), in which Xaa is any amino acid but Proline, into various sites of SC may allow for selection of functionally active variants with improved characteristics (e.g. stability, binding to pathogen structures, binding to pIg).

The variant of the polypeptide or the nucleotide sequence is functionally active in the context of the present invention, if the activity of the composition of the invention including the variant (but not the original) amounts to at least 50%, preferably at least 60%, more preferred at least 70%, still more preferably at least 80%, especially at least 90%, particularly at least 95%, most preferably at least 99% of the activity of the immunoglobulin or SC as used according to the invention including the amino acid or the nucleotide sequence without sequence alteration (i.e. the original polypeptide or the nucleotide sequence).

Functionally active variants may be obtained by changing the sequence as defined above and are characterized by having a biological activity similar to that displayed by the respective sequence from which the variant is derived or similar to human Slg, including the ability of supplement an SIgA or SIgM deficiency in the mucosa, and/or to modulate the immune response to antigens, the binding to pathogen structures, receptors and other molecules.

A functionally active Slg variant may be obtained by exchange of domains between Slg from different species, or the use of chimeric sequences. e.g. J-chain from chicken may be combined with Ig from mouse and this molecule may be complexed with secretory component of human origin. All kinds of combinations of the various polypeptide chains that make up Slg (composed of light chain, heavy chain, J-chain and secretory component) can be employed. The various chains may be recombinant and/or non-recombinant molecules, they may be chimeric (such as e.g. mouse/human heavy and light chains) or otherwise modified.

Still, the term "functionally active variant" includes naturally occurring allelic variants, as well as mutants or any other non-naturally occurring variants. As is known in the art, an allelic variant is an alternate form of a (poly)peptide that is characterized as having a substitution, deletion, or addition of one or more amino acids that does essentially not alter the biological function of the polypeptide.

In a preferred embodiment, the functionally active variant derived from the amino acid or the nucleotide sequence as defined above by amino acid exchanges, deletions or insertions may also conserve, or more preferably improve, the activity.

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

The term "mucosal immunoglobulin deficiency" or "mucosal Ig deficiency" shall mean a concentration in immunoglobulins present in mucosal samples below the normal or reference range. This specifically refers to the content of either SIgA or SIgM or a combination of both SIgA and SIgM.

The term "secretory IgA deficiency" or "SIgA deficiency" is a deficiency in secretory IgA. The term "secretory IgM deficiency" or "SIgM deficiency" is a deficiency in secretory IgM.

These secretory immunoglobulin deficiencies may be caused by genetic disposition, chemicals, or by local disturbance of Slg production in the mucosa-associated lymphoid tissue (MALT) e.g. by stress, malnutrition, injury or surgery. MALT is understood in man as e.g.
- GALT (gut-associated lymphoid tissue. Peyer's patches are a component of GALT found in the lining of the small intestines.)
- BALT (bronchus-associated lymphoid tissue)
- NALT (nose-associated lymphoid tissue)
- LALT (larynx-associated lymphoid tissue)
- SALT (skin-associated lymphoid tissue)
- VALT (vascular-associated lymphoid tissue)
- EALT (eye-associated lymphoid tissue made up of Conjunctiva [CALT] and Lacrimal Duct [LDALT] associated lymphoid tissues)

SIgA deficiency may be associated with SIgM deficiency.

Detection of secretory IgA and/or secretory IgM deficiency is done by measurement of SIgA and/or SIgM in secretions such as saliva, cervical mucus, nasal mucus, gastric juice, sweat, urine or stool by standard immunoassay techniques or by molecular biology techniques. Immunological detection of SIgA and/or SIgM in secretions can be performed by ELISA, RIA, by fluorescence based immune assays, time-resolved fluorometry, precipitation assays, nepheliometric assays, surface plasmon resonance based assays and similar setups with and without labels. An important requirement is the detection of the Secretory Component bound to the immunoglobulin molecules in order to be able to discriminate SIgA and/or SIgM from IgA and/or IgM.

Molecular biological detection of SIg deficiency can be performed by assaying for the expression of J-chain and/or pIgR in respective tissues and cells, either at the protein level with antibodies, specific for those molecules or on the genetic level with probes specific for the genes of those molecules. Both RNA and DNA may be investigated. Methods for detection can be assays based on hybridization with or without template amplification (such as PCR) or signal amplification. Alternatively, it is possible to test for risk factors for IgA deficiency: MHC related genes (e.g. HLA-A1, B8, DR3, DQ2(8.1)), and IFIH1 and CLEC16A,all of which have been reported to be associated with IgA deficiency.

A mucosal SIgA deficiency may be a strong indicator of general secretory IgA deficiency. A mucosal SIgM deficiency may be a strong indicator of general secretory IgM deficiency. A combined mucosal SIgA/SIgM deficiency is a strong indicator of a combined general secretory IgA and IgM deficiency.

An SIg deficiency is particularly indicated if the level of mucosal SIg is less than 50% as compared to a reference value, which is either a normal value or a value of healthy subjects of the same type, specifically less than 40%, 30%, 20% or 10%.

There is considerable variation in the levels of secretory immunoglobulins in different individuals. An exact measurement of the immunostatus of an individual may first establish the baseline of usual SIgA and/or SIgM values for the individual over a period of days to weeks during a period of good health and low to moderate physical activity. The baseline may also vary with the age of the individual. However this method is not feasible for individuals with chronic secretory immunodeficiency. Therefore, reference values for normal populations may be considered as well.

For humans, the normal salivary SIgA level is 11-65 mg/dL, normal salivary SIgM level is 1 mg/dL. Normal SIg values are typically determined in samples of healthy subjects. Decreased salivary Ig may be present in children with recurrent upper respiratory infection, selective IgA and/or IgM deficiency and occasionally in individuals with food allergies.

SIgA deficiency in human saliva samples as determined by immunoassays is typically indicated if the SIgA concentration is less than 100 milligram SIgA per liter or a salivary SIgA flow rate of less than 50 micrograms SIgA per minute or an SIgA to albumin ratio of less than 4 (as proposed by Dwyer et al. in Aviation, Space, and Environmental Medicine, 2010, volume 81, pages 582 ff). SIgA-deficiency in human faeces as determined by immunoassays is typically indicated if the SIgA concentration is less than 10 milligram per 100 g of faeces.

SIgA deficiency in human tears as determined by immunoassays is typically indicated if the SIgA concentration is below 50 mg SIgA per milliliter.

SIgM deficiency in human nasal secretions or saliva as determined by immunoassays is typically indicated if the SIgM concentration is less than 50 mg per liter in nasal secretions and/or an SIgM concentration is less than 1 mg/dL in saliva.

The decreased level of SIg needs to be found in only at least one compartment of the mucosa-associated lymphoid tissue in order to qualify for SIg deficiency.

SIgA deficiency occasionally may be determined in a sample wherein an SIgM deficiency was also determined. Thus, the preparation of the present invention comprising both SIgA and SIgM is preferably used in a subject at risk of SIgA and SIgM deficiency.

The term "mucosal" with respect to an immunoglobulin deficiency refers to the level of the immunoglobulin as determined in mucosal samples, such as samples taken from a subject's saliva, gastric juice, cervical mucus, nasal mucus, gut lavage, gastric juice, bronchial lavage, urine, tears and faeces.

The term "mucosal" with respect to administration or application or else mucosal use of a preparation for treating a subject or a respective formulation, refers to administration via the mucosal route, including systemic or local administration, wherein an active ingredient is taken up by contact with mucosal surfaces. This includes oral, nasal, vaginal, rectal administration and formulations, e.g. liquid, syrup, lozenge, tablet, spray, powder, instant powder, granules, capsules, cream, gel, drops, suspension, emulsion or food product.

The term "subject" as used herein refers to any animal, which herein preferably includes mammals and particularly human, for whom diagnosis, screening, monitoring or treatment is contemplated. A subject may be at risk of a certain disease condition, e.g. a patient afflicted with a disease condition or for which a disease condition is to be determined or risk of a disease condition is to be determined. The term "patient" as used herein always includes healthy subjects. In some embodiments, the methods disclosed herein may include selecting a subject in need of an SIg supplemental therapy, such as a subject with proven SIg deficiency, and further treating said subject according to the invention.

The term "at risk of" a certain disease conditions, such as SIg deficiency or mucosal immunoglobulin deficiency refers to a subject that potentially develops such a disease condition, e.g. by a certain predisposition, or already suffers from such a disease condition at various stages, including the congenital or acquired state, including transient disease, particularly associated with other causative disease conditions or else conditions or complications following as a consequence of such immunoglobulin deficiency.

The risk determination and diagnosing a mucosal Ig deficiency is particularly important in a subject, where the Ig deficiency has not yet been diagnosed. This risk determination therefore includes early diagnosis to enable prophylactic therapy. Risk assessment may be performed by single, preferably by multiple risk parameters such as genetic background, stress level, ingestion of certain drugs etc.

Specifically the preparation of the invention is used in patients with a high risk, e.g. a high probability of a mucosal Ig deficiency without symptoms (e.g. children below 4 years, persons shortly before and after surgery, before and after extensive physical stress such as high performance sports or work, or when traveling with an increased risk of infection with pathogens).

The risk assessment and in particular the treatment of mucosal Ig deficiency according to the invention is particularly indicated with infectious diseases of the mouth, throat, nose and ears, the eyes and esophagus, the gastric and colon tract.

A further preferred use is the prevention of a disease condition caused by a pathogen, including microbial substances or organism, antigens or disease-causing agents, such as toxins. For example, hospitalised patients may need to supplement their immune system to reduce the risk of a hospital-acquired infection. Neonatal humans or animals receiving a food supplement according to the invention may have a higher chance of survival in case that they cannot obtain sufficient breast milk from mothers or respective wet nurses. Furthermore, the risk of enteropathogenic disease in animals and humans may be reduced by a food product according to the invention. Specifically, where a subject is suffering from SIg deficiency, pathogens may induce a hypertoxic effect, i.e. a disease condition upon challenging with a dose of a disease causing agent, which would otherwise not cause such disease condition. The preparation according to the invention is, thus, specifically provided to prevent such hypertoxic effect in subjects at risk of or suffering from SIg deficiency.

The term "secretory immunoglobulin" and "Slg" as used herein shall specifically refer to either of SIgA or SIgM or the sum of SIgA and SIgM, also referred to as "SIgA and/or SIgM", which is interchangeably used with the term "SIgA/SIgM".

The term "single-dose" as used herein is understood in the following way. A single-dose or amount for single-use is the amount intended for administration that is meant for use in a single subject, such as a patient, either human or animal for a single case/procedure/administration. Packages comprising the single-dose are typically labelled as such by the manufacturer. The single-dose amount is specifically understood as a daily dose for an individual, like a child or adult, to provide an effective amount.

The term "supplement" with respect to Slg deficiency as used herein refers to treating such deficiency by administering Slg in an effective amount to establish at least 50% of a reference SIgA level, e.g. from a healthy subject, or a normal value, preferably at least 60%, 70%, 80%, 90% or at least 100%. It may also be necessary to supplement Slg at a dose to establish relatively high mucosal IgA or IgM concentrations of at least 150%, 200% or even more, e.g. as a bolus or employing high dose formulations. Relatively high concentrations are specifically indicated in high risk situations.

The term "supplement" with respect to Vitamin A as used herein refers to addition of molecules with Vitamin A activity to a preparation which may or may not contain any of such molecules. In a composite preparation based on purified components, the Vitamin A supplement is understood as a purified additive to an Slg preparation, which is obtained by enrichment and purification from natural sources, such as milk or milk fraction, or else obtained from cell cultures or as recombinant proteins. Exemplary Slg preparations are e.g. prepared using milk or milk fractions of mammals as a source material, which may have a natural content of Vitamin A. In this case the Vitamin A supplement refers to an addition of molecules with Vitamin A activity that are different from the Vitamin A molecules of the source material. In addition or alternatively, the supplement would typically provide for an increased amount of Vitamin A or Vitamin A activity in the SIg preparation obtained from such source material, e.g. at least 120%, preferably at least 130%, or at least 140%, or at least 150%, or at least 160%, or at least 170%, or at least 180%, or at least 190%, or at least 200%, or even more, as compared to the average amount in a preparation obtained from the source material. For example, if milk or milk fractions is used as a source material for preparing the SIg preparation, the Vitamin A supplement comprises either compounds with Vitamin A activity, different from beta-carotene, retinol and retinol esters, and/or Vitamin A in an amount of at least 100 or at least 200, or at least 500 or at least 1000 retinol activity equivalents per 100 g of product, or more than 2 retinol activity equivalents (RAE) per mg SIg, preferably more than 5 retinol activity equivalents per mg SIg, most preferably more than 10 retinol activity equivalents per mg Slg.

The term "treatment", "treating" or "therapy" as used herein with respect to treating subjects refers to medical management of a subject with the intent to cure, ameliorate, stabilize, reduce the incidence or prevent a disease, pathological condition, or disorder, which individually or together are understood as "disease condition". The term includes active treatment, directed specifically toward the improvement of a disease condition, prophylaxis directed specifically toward the prevention of a disease condition, and also includes causal treatment directed toward removal of the cause of the associated disease condition. In addition, this term includes palliative treatment designed for the relief of symptoms rather than the curing of the disease condition, and further curing a disease condition directed to minimizing or partially or completely inhibiting the development of the associated disease condition, and supportive treatment employed to supplement another specific therapy directed toward the improvement of the associated disease condition.

The term "effective amount" as used herein refers to such amount as is capable of performing the function of the compound or property, for which an effective amount is expressed. As will be pointed out below, the exact amount required will depend on the subject in need of a therapy or the respective disease condition to be treated. An appropriate effective amount may be determined by a skilled person using only routine experimentation. In specific aspects, an amount can be therapeutically effective, e.g. to treat an existing disease condition. In further aspects, a preparation can be prophylactically effective, e.g. for prevention of a disease condition. In a further aspect, an SIg compound, Vitamin A compound or preparation according to the invention may be provided in a dose effective to supplement mucosal Ig deficiency.

The term "formulation" as used herein refers to a preparation ready-to-use for treating a subject in a specific way. Formulations according to the invention may be mucosal formulations for administration via the mucosal route or else enteral or topical formulations.

The term "Vitamin A" as used herein is understood as a molecule with Vitamin A activity, as determined in a RAR (retinoic acid receptor) or RXR (retinoid X receptor) agonistic activity assay. The Vitamin A activity may be measured as retinol activity equivalent (RAE) according to the US Institute of Medicine. Each µg RAE corresponds to 1 µg retinol, 2 µg of β-carotene in oil, 12 µg of "dietary" beta-carotene, or 24 µg of the three other dietary provitamin-A carotenoids (alpha-carotene, gamma-carotene, beta-cryptoxanthin). Vitamin A in the form of retinol is an essential micronutrient required in the diet of all vertebrates. Vitamin A is long been known as a requirement for the maintenance of normally differentiated epithelial cells, through the regulation of cell turnover, trafficking and mucin production. It is understood that the term particularly refers to naturally occurring molecules, including natural forms of Vitamin A and functionally active derivatives.

A subgroup of common variable immunodeficiency (CVID) patients has been characterized with low Vitamin A levels. It was shown that the supplementation of Vitamin A to CVID patients with initial low Vitamin A levels improved serum IgA levels.

The group of molecules with Vitamin A activity specifically includes retinal, retinol, retiniods, retinoic acids (such as all trans-retinoic acid, 13-cis-retinoic acid), retinylpalmitate, 3-dehydroretinol, 3-dehydroretinal, beta-carotene, alpha-carotene, gamma-carotene, and beta-cryptoxanthine. Specifically included in the list of molecules with Vitamin A activity are also functionally active variants of such molecules. A preferred combination for oral use is SIgA and retinylpalmitate, specifically comprising 10 mg to 10 g SIgA and 100 to 5000 micrograms RAE, per dose.

Vitamin A is typically administered at a dose of 30-3000 micrograms per day e.g. for treating a human being. The preferred amount of RAE is 100 to 5000 micrograms, more preferred is 500 to 2000 micrograms. Preferred preparations according to the
invention employ Vitamin A, preferably retinylpalmitate, at a dose of 800 micrograms or about 800 micrograms, e.g. within the range of 700 to 900 micrograms, to provide an effective amount or Vitamin A activity. An effective amount of beta-carotene may be more than 50 micrograms per day. All-trans-retinoic acid typically would be given at a dose of 1-2000 micrograms per day.

All-trans-retinoic acid (RA), one of two major metabolites of retinol, is a potent regulator of cell proliferation and differentiation, with pleiotropic effects, due to regulation of gene expression, in essentially all organ systems. The genomic actions of RA are mediated by its binding to transcription factors of the RA nuclear receptor family RAR (RARalpa, beta and gamma), while 9-cis-RA binds to RXR receptors.

The high susceptibility of infants and neonates to infections, and their generally weaker response to vaccination as compared to older children and adults, is mostly attributed to the relative immaturity of the immune system and a relative Vitamin A deficiency. It has been shown that beta-carotene supplementation for maternal mice during pregnancy and lactation is useful for enhancing IgA transfer from maternal milk to neonates owing to the increase in IgA secreting cells in mammary gland and ileum during lactation. All-trans retinoic acid (RA), plays important roles in gut immunity and it is necessary for the imprinting of gut-homing specificity on T cells and the induction of guthoming receptors on B cells and IgAsecreting cells (Br. J. Nutr. 2011, volume 105, pages 24-30).

Vitamins and minerals have often been described to have an influence on the immune system. Some of these substances influence in a general way the immune system, whereas some seem to be more specific. It has been described that SIg preparations (milk fractions) may be combined with various vitamins and minerals, however, no specific effect was anticipated in view of the prior art.

It has been shown that a Vitamin A supplementation of man with 20 mg beta carotene per day did not increase the saliva secretion of SIgA as compared to the placebo group (International Journal of Vitamin and Nutrition Research, 1988, volume 58 pages 171 ff).

Therefore, the present invention refers to an improved SIg preparation or combination of SIg with Vitamin A for use in treating subjects at risk of mucosal Ig deficiency, specifically for the therapy or prophylaxis of mucosal immunoglobulin deficiency. It surprisingly turns out that SIg and Vitamin A act synergistically to supplement Ig deficiency, e.g. as determined in a mucosal SIg recovery assay with *in vivo* samples. Thus, it was the first time that an agonist of a retinoid receptor, such as Vitamin A, was found to act together with SIgA for an improved supplementation therapy. Vitamin A as a retinoid receptor agonist is specifically capable of binding either a retinoid X receptor or a retinoic acid receptor of a cell and triggers a response by that cell.

The retinoic acid receptor (RAR) is a type of nuclear receptor that is activated by both all-trans retinoic acid and 9-cis retinoic acid. There are three retinoic acid receptors (RAR), RAR-alpha, RAR-beta, and RAR-gamma, encoded by the RARA, RARB, RARG genes, respectively. Each receptor isoform has several splice variants: two- for alpha, four- for beta, and two- for gamma.

RAR heterodimerizes with RXR and in the absence of ligand, the RAR/RXR dimer binds to hormone response elements complexed with corepressor protein. Binding of agonist ligands to RAR results in dissociation of corepressor and recruitment of coactivator protein that, in turn, promotes transcription of the downstream target gene into mRNA and eventually protein.

The retinoid X receptor (RXR) is a type of nuclear receptor that is activated by 9-cis retinoic acid. 3 retinoic X receptors (RXR) have been described: RXR-alpha, RXR-beta, and RXR-gamma, encoded by the RXRA, RXRB, RXRG genes, respectively.

RXR heterodimerizes with subfamily 1 nuclear receptors including CAR, FXR, LXR, PPAR, PXR, RAR, TR, and VDR. The RXR heterodimer in the absence of ligand is bound to hormone response elements complexed with corepressor protein. Binding of agonist ligands to RXR results in dissociation of corepressor and recruitment of coactivator protein, which, in turn, promotes transcription of the downstream target gene into mRNA and eventually protein.

In a specific preparation according to the invention the immunoglobulins are polyclonal, multispecific and/or polyreactive monoclonal antibodies, including crossreactive antibodies. This has the advantage of providing natural antibodies or supporting the subject's innate immune system. The preparation may contain 1, 2, 3 or even more different monoclonal antibodies in a mixture or as a set, of which at least one of the antibodies shows polyreactivity.

Specifically the preparation according to the invention comprises a polyreactive immunoglobulin, preferably a natural immunoglobulin, including germline antibodies.

Specifically the preparations are provided comprising multispecific antibodies or a mixture of antibodies specifically targeting only one antigen, or targeting more than one antigen, e.g. at least two antigens or at least three antigens. For example, preferred targets are epitopes of pathogens, such as Botulinum neurotoxins (BoNTs), Anthrax toxin, Subtilase cytotoxin (SubAB), Pasteurella multocida toxin (PMT), Vibrio Multifunctional-Autoprocessing RTX toxins, Helicobacter VacA, Staphylococcus toxins, mycotoxins such as aflatoxins, ochratoxin, citrinin, patulin, ergotamine, cytochalasin, fusarium toxins, fumonisin, gliotoxin, muscimol, phalloidin, sterigmatocystin, trichothecene, vomitoxin, zeranol, ribotoxins, cholera toxin, cyanotoxins, diphteria toxin, *E. coli* produce heat-stable enterotoxins (ST), perfringolysin, pneumolysin, listeriolysin, HlyA hemolysin, alpha-toxin of C. perfringens, AB-toxins such as pertussis toxin, cholera toxin, shiga toxin, heat labile enterotoxin of *E. coli,* pseudomonas exotoxin, lipopolysaccharides, lipooligosaccharides, peptidoglycan, lipoteichoic acid, keyhole limpet hemocyanin, tetanospasmin, *C. difficile* toxins A and B, leukocidin, exfoliatin, toxic shock syndrome toxin, cord factor, veratoxin, shiga-like-toxin, extracellular adenylate cyclase, clumping factor A, fibronectin binding protein A, noroviruses, norovirus proteins rotavirus, rotavirus proteins, influenza virus, influenza virus proteins, influenza hemagglutinin, influenza neuraminidase, *E. coli* type I fimbriae, *Streptococcus mutans* Cell surface protein antigen (Pac), *Candida albicans,* EPEC intimin, EHEC hemolysin, *Streptococcus pneumoniae* choline-binding protein A, ricin toxin, *Helicobacter pylori, Salmonella typhimurium,* Salmonella sp., *Shigella, reovirus,* flagellin, *Lactobacillus rhamnosus, Bifidobacterium lactis, Bacteroides thetaiotaomicron, H. pylori* BabA adhesin, *H. pylori* SabA, *H. pylori* AlpA, AlpB and HopZ and *Hemophilus influenzae.*

It is preferred that the preparation according to the invention comprises a high titre of a relevant mix of immunoglobulins maintaining their native function including the mucosal passage after oral ingestion.

Specific preparations of the invention comprise human polyclonal or polyreactive Slg, e.g. with a specific IgA2:IgA1 ratio of at least 2:1, preferably at least 5:1. Specific preparations of the invention comprise polyclonal or polyreactive Slg with a specific IgM:IgG ratio of at least 1:1, preferably at least 2:1, more preferably at least 5:1, most preferably at least 10:1.

The preparation according to the invention specifically comprises a polymeric immunoglobulin, such as a dimeric, or pentameric or other polymeric immunoglobulin, e.g. dimeric SIgA and/or pentameric SIgM.

The preparation according to the invention may further comprise free, unbound SC besides SC complexed with immunoglobulin, such as SIgA or SIgM, so to provide a respective SIgA complex or SIgM complex, to enhance its functional properties.

A preferred embodiment of the invention employs an SC molecule attached to a natural antibody in order to provide a proteolytically stable, multivalent and polyspecific molecule that is able to neutralize various pathogen antigens.

It is further preferred that the preparation according to the invention contains from 5 to 100% (w/w protein) secretory immunoglobulins. More particularly, the preparation may contain from 20 to 70% (w/w protein) secretory immunoglobulins.

It is preferred that the SIg is employed as immune complex with SC comprising a human-like N-glycosylation pattern. Specifically, a preparation according to the invention comprises SIgA with Lewis-type glycosylation (antennary fucosylation) of the secretory component. Suitable industrial production systems would employ source materials from pooled sources of mammary gland secretions, recombinant cell cultures and blood plasma, which preferably provide for the desired glycosylation pattern.

It is preferred that the production system is capable of producing Lewis-type N-glycosylated proteins, specifically the secretory component with peripheral or antennary, such as outer arm, fucosylation as determined by suitable analytical means, such as electrophoretic, chromatographic, mass spectroscopic, chemical and enzymatic techniques or combinations thereof.

In a specific embodiment said immunoglobulin is derived from an amino acid or nucleotide sequence of a species such as human, cow, goat, sheep, non-human primate, pig, mouse, rat, rabbit, dog, wallaby, possum, panda, fish, chicken, bird, frog, or chimeric sequences thereof. Preferably the immunoglobulin is derived from an amino acid or nucleotide sequence of mammalian origin, specifically human, cow, goat, sheep, non-human primates, pig, camel, dromedary, donkey or horse, or chimeric sequences thereof.

In a specific embodiment said immunoglobulin is a combination of pIg and SC, wherein both may stem from the same or different species, both may be recombinant or natural proteins or a mixture of recombinant and natural. (e.g. pIg from plasma and recombinant SC).

In a preferred method the SIg is enriched in a fraction obtained from a suitable industrial production system, such as milk and optionally isolated from said fraction. Specifically said SIg is obtained from a source, e.g. by isolation techniques, which source is a fraction of the production system enriched in said SIg, such as milk and optionally isolated from said fraction.

According to a specific method, said SIg is obtained from pooled sources selected from the group consisting of milk, milk concentrates, milk powders, whey, whey concentrates, whey protein concentrates, whey protein isolates and whey powders, derived from at least 10 female individuals in the lactating phase.

Specifically said individuals are of a species selected from the group consisting of human, goat, cow, buffalo, sheep, horse, donkey, dromedary, pig and camel.

Preferably said individuals are selected from a population or herd for the capability of expressing native N-glycosylated SIgA or SIgM with Lewis-type glycosylated secretory component. Such individuals would produce "human-like" Slg.

Selections are made, for instance according to the capability of the individual to produce pIgR and secretory component with Lewis-type N-glycosylation. According to the invention it is specifically preferred that the amount of Lewis epitopes amounts to at least 10% of the theoretical value, preferably at least 20%, 30%, 40%, 50%, 60%, 70%, 80% or 90% up to the theoretical value as calculated from the number of N-glycosylation sites on the secretory component.

Specifically, the Secretory Component of the invention comprises sialic acid, preferably at least 2 mol sialic acid per mol Secretory Component. More specifically the SC comprises at least 2 mol sialyl Lewis x per mol SC.

Preferred production systems are derived from goat, e.g. to provide a goat milk fraction as source material, such as a whey fraction. A preferred source material is a pool of goat milk fractions, selected for its specific glycosylation pattern.

A more preferred production system is pig, e.g. to provide sow milk as a source material, selected for its specific N-glycosylation pattern of the secretory component.

A preferred source material is blood plasma or a fraction thereof containing polymeric immunoglobulin (such as dIgA and IgM). Such plasma or serum derived polymeric immunoglobulin may be processed to form secretory immunoglobulin complexes with J-chain and secretory component from either natural or recombinant sources.

According to a specific embodiment said Secretory Component is obtained from a recombinant host cell expressing autologous or heterologous N-glucosyltransferases and especially fucosyltransferases preferably a recombinant production host cell line expressing autologous or heterologous functional alpha-1,x-fucosyltransferase, wherein x is 2,3 or 4, preferably selected from the group consisting of human cell line, mammalian cell line, avian cell line, bacteria, plant, yeast, insect, fungal, moss and archaea.

Preferably the host cell is selected from the group consisting of human cells such as PerC.6, Chinese Hamster Ovary cells, Baby Hamster Kidney cells, murine cells, avian cells lines, bacterial, yeast fungal, plant and insect cells. Thereby a recombinant SIgA or SIgM may be obtained with the desired Lewis-type N-glycosylation.

The elucidation of the interactions between pathogen structures such as surface antigens or toxins and glycan structures of the host may lead to preparations according to the present invention with improved functional bioactivity. In particular, Lewis glycosylation may interact with *Helicobacter pylori* attachment to host cells. More than 80% of *H. pylori* strains express Type II Lewis antigens (LeX and/or LeY), and half of them express both. A smaller proportion of *H. pylori* strains express Type I Lewis blood group antigens (LeA and/or LeB) and a very small number express sialyl-LeX.

Similarly, sialyl-LeX is expressed on the cell surface of some oral bacteria that are associated with infected endocarditis, such as *Streptoccocus pyogenes, Porphyromonas gingivalis, Actinobacillus actinomycetemcomitans* and *Eikenella corrodens.*

Noroviruses are causative agents of e.g. acute gastroenteritis. They bind to histo-blood group antigens (HBGAs) on host cells, namely, ABH antigens and Lewis antigens, in which type 1 and type 2 carbohydrate core structures constitute antigenically distinct variants. Human noroviruses recognize sialyl Lewis x neoglycoprotein.

*Clostridium difficile* toxin A and intimin of enteropathogenic E.coli are binding to galactosyl and/or sialic acid residues of SC.

The invention particularly provides for preparations with high potency for use in pathogen caused disease conditions. Standard potency testing typically refers to the neutralisation activity or binding assays of at least one of *Clostridium difficile* toxin A, *Helicobacter, E.coli* toxins, *Campylobacter, Shigella,* Rotaviruses, competition, binding inhibition or other interactions with lipopolysaccharides, lipoteichoic acid, peptidoglycan and keyhole limpet hemocyanin.

A human-like glycosylation of an SC can be found in selected individuals of non-human species, and that such human-like SC is preferentially used in combination with a native immunoglobulin that is useful to stabilize the Slg as used according to the invention.

The glycosylation pattern of SC or immunoglobulin may vary within the same species in a broad range, thus, large pools of donations that are typically used for preparing milk products would not contain a significant level of N-glycosylation with Lewis-epitopes on the isolated SC. Such common large scale pools are preferably not used as source material for the purpose of the invention. In some individuals, however, the SC can have human-like, e.g. high level of Lewis epitopes. Those individuals would qualify to prepare large production pools that can be used as a source material for manufacturing the Slg immune complex as used according to the invention at large scale. Alternatively the respective human-like SC may be produced by recombinant production methods. The SC obtained from the source material is then combined with IgA and/or IgM molecules having a native glycosylation pattern. Alternatively, immune complexes of the invention comprising the SC and the immunoglobulin can be directly isolated and purified from large production pools, e.g. selected for their content of Lewis epitopes. Thus, the Slg immune complex preparation may be prepared, which comprises the stabilized SC to the extent that it provides for the storage-stable product or immune complex preparation with an increased stability *in vivo,* resulting in an increased recovery in the mucosa and/or half-life.

Glycosylation variations may occur at a different physiological state of the cell or organism producing the SC or immunoglobulin, e.g. infections, presence of inflammatory factors, food and nutrition supply, stress etc. A further source of variation of glycosylation in a mixture may be a different genetic background and makeup of the individual organisms or cells producing the compounds (such as species, race, blood groups etc.). Suitable analytical methods to determine the glycosylation pattern are e.g. described by Deshpande et al. (J. Proteome Res. 2010, 9, 1063-1075).

Source animals may be prescreened for the likelyhood to produce more of SIgA and/or SIgM or more of a certain glycosylation by genetic means, e.g. by assays for expression of pIg-receptor and/or certain fucosytransferase genes, e.g. FUT3, 4, 5, 6, 7, 9. 10, 11 and/or other glycosyltransferases (e.g. beta-3-galactosyltransferase or beta-4-galactosyltransferase) and a population may be bred for such purposes.

Such animals may then be identified and selected for the suitable expression products. Testing may be performed by immunological assays specific for Lewis-epitopes employing specific antibodies or receptors (such as DC-SIGN).

The expression product comprising the Lewis-fucoslated SC or immunoglobulin as described above is then preferably pooled and may be used as a source for the preparation according to the invention.

The production pools, specifically those having a proven high quality glycosylation profile, have a preferred size of at least 10, more preferred at least 50, 100, 500, 1.000 or 2.000 donations, or preferably at least 10, 50, 100, 500, 1000, 2000, 10,000 or 20,000 Liters.

Preferably the donor expression system is comprised of non-human, female individuals in the lactating phase to obtain the SIg from the donors' milk or milk fractions. Exemplary source materials are e.g. whey, or dried whey, which contain Slg, polymeric immunoglobulins and immune complexes, respectively, in the enriched form. Preferred source material may be at least 2 fold, 3 fold, 4 fold or 5 fold enriched in immunoglobulins.

Immunoglobulins and specifically the SIgA and/or SIgM may then be obtained from such source material in the purified form for use in a preparation according to the invention.

Expression systems alternatively used for producing the SIg according to the invention may also be recombinant expression systems, among them recombinant host cells of all species and taxa, e.g. recombinant eukaryotic hosts.

Therefore, an amino acid sequence or nucleotide sequence coding for an SIg or part thereof, such as the SC or IgA or sIgM, may be employed to prepare a recombinant host. The sequences preferably encode polypeptides of mammalian origin, such as human, cow, goat, pig, sheep or humanized versions of non-human sequences, or chimerics, always including functional variants. Respective sequence information may be derived from public databases, as appropriate.

Recombinant SIgA or SIgM to date has been produced in hosts that are unable to add Lewis-type fucosylation to the polypeptides. Hosts for purified recombinant SIgA or SIgM or parts thereof that were commonly used were CHO cells, BHK cells, mouse J558L cells, insect cells and tobacco plants.

It is specifically preferred that expression systems are used which are able to glycosylate proteins in the desired way, *per se,* i.e. in the hereditary way, or else by the acquired or transient capability through respective genetic modifications, e.g. through recombination techniques to provide for the respective glycosylation pattern. Exemplary expression systems have an enhanced capability to produce Lewis-fucosylated pIgR, e.g. through the concomitant expression of fucosyltransferase 2 and 3 and beta 1,3-galactosyltransferase I, II and V or variants thereof.

Hosts that are engineered to produce Lewis-glycosylated oligosacharides and glycoconjugates are, for instance, production cell lines to express various glycosyltransferases such as fucosyltransferases in order to produce oligosaccharides, glycoproteins or glycolipids with blood group related antigens.

Engineered CHO cells have been described to generate Lewis type fucosylation (Löfling et al. 2008, Glycobiology vol. 18 no. 7 pp. 494-501). However, expression of complete SIg in such host cells would lead to Lewis-fucosylated alpha-immunoglobulin chains with non-native glycosylation. Such cells may therefore be used only to produce human-like SC, after selection of appropriate glycosylation according to the invention. Selection of appropriate recombinant host cell clones is performed as described for the screening of milk samples as described above. It is preferred to engineer, screen and select for more different Lewis-type glycosylations (such as Lewis x, Lewis y, Lewis a and sialylated forms thereof) than described by Löfling et al. Recombinant polypeptides derived from such host cells may be used in a preparation according to the invention, e.g. a recombinant Lewis type fucosylated SC in combination with a (recombinant or non-recombinant) immunoglobulin from other sources.

Specifically it is preferred to cultivate a recombinant host cell line in a bioreactor on a pilot or industrial scale employing conditions to express Lewis-glycosylated SC with yields of at least 1 mg/L culture medium, preferably at least 10 mg/L, preferably at least 100 mg/L, most preferred at least 1 g/L.

The host cell according to the invention is preferably tested for its expression capacity or yield by the following test: ELISA, activity assay, HPLC, or other suitable tests which show the amount and quality of SC according to the invention. The host cell is selected not only for expression levels but also for the glycosylation pattern of the SC it is able to provide: e.g. at least one of Lewis a, Lewis b, Lewis x and Lewis y or its sialylated forms are to be found on the SC. Preferably, Lewis x and/or sialyl-Lewis x is present in a sample. More preferable, more than one type of Lewis-antigens on SC is present in a sample. A specifically preferred SC preparation comprises at least 2 mol sialyl-Lewis x epitopes per mol SC, in some cases at least 6 mol/mol. Preferably the SC as provided in such preparation contains either only asialylated glycans or sialylated glycans.

Preferred fermentation techniques are batch, fed batch or continuous cultivation such as perfusion culture.

Preferably the production cell line is cultivated in a mineral medium with a suitable carbon source, thereby further simplifying the isolation process significantly. An example of a preferred mineral medium is one containing an utilizable carbon source (e.g. glucose, glycerol or methanol), salts containing the macro elements (potassium, magnesium, calcium, ammonium, chloride, sulphate, phosphate) and trace elements (copper, iodide, manganese, molybdate, cobalt, zinc, and iron salts, and boric acid), and optionally vitamins or amino acids, e.g. to complement auxotrophies.

The transformed cells are cultivated under conditions that are suitable to effect expression of the products which can be purified from the cells or culture medium, depending on the nature of the expression system. As will be understood by the skilled person, cultivation conditions will vary according to factors that include the type of host cell and particular expression vector employed.

If the products are secreted from the cells, they can be isolated and purified from the culture supernatant using state of the art techniques. Secretion of the recombinant expression products is generally advantageous for reasons that include facilitating the purification process, since the products are typically recovered from the culture supernatant rather than from the complex mixture of proteins that results when cells are disrupted to release intracellular proteins.

The cultured transformant cells may also be ruptured sonically or mechanically, enzymatically or chemically to obtain a cell extract containing the desired product, from which the product is isolated and purified.

Isolation and purification methods used for obtaining an SC or SIg as used according to the invention may utilize differences in solubility, such as salting out and solvent precipitation, differences in molecular weight, such as ultrafiltration and gel electrophoresis, differences in electric charge, such as ion-exchange chromatography, or may utilize specific affinities, such as affinity chromatography, or may utilize differences in hydrophobicity, such as reverse phase high performance liquid chromatography, or utilize differences in isoelectric point, such as isoelectric focussing. Specific purification steps that are preferably employed to separate any SC or immunoglobulin polypeptides alone or in complex with other compounds are ultrafiltration techniques with molecular cutoffs between 100 kDa and 500 kDa and precipitation techniques such as precipitation with salts such as ammonium sulfate or organic solvents.

The isolated and purified products can be identified and analysed by conventional methods such as Western blotting or assay of its activity, e.g. by its ability to bind to other components of the SIg complex, such as SC, IgA, dimeric IgA or the J-chain, IgM, pentameric IgM, or by detection with specific antisera.

The structure of the purified compound can be defined by amino acid analysis, amino-terminal analysis, primary structure analysis, glycoanalysis and the like. It is preferred that the products are obtainable in large amounts and in specific cases with a high purity, thus meeting the necessary requirements for being used as active ingredient in pharmaceutical compositions.

According to a specific embodiment there is provided a formulation comprising a preparation according to the invention, in the form of a liquid, emulsion or suspension, slurry or in the dried form, preferably spray-dried or freeze-dried. The preferred preparation is in a ready-to-use, storage stable form, with a shelf life of at least one or two years.

Specifically preferred formulations are manufactured as a powder or granulate which can be formulated into a liquid instantly before use.

Further preferred administration forms are tablets, lozenges, capsules, pastes, granules, creams, gels, drops, emulsions, suspensions, chewing gums, sprays etc. which may be produced by standard methods. Tablets preferably contain auxiliary additives such as fillers, binders, disintegrants, lubricants, flavors or the like). Granules may be produced using isomaltose. It is furthermore preferred to provide for a preparation formulated to act at the site of the mucosa, e.g. at mucosal sites (nose, mouth, eyes, esophagus, throat, lung, ears, gastric tract, vagina, penis, intestine, rectum and colon), e.g. locally without systemic action. The preparation according to the invention typically is provided for oral or mucosal use, including oral, nasal, vaginal, rectal use, e.g. to inhibit adherence to pharyngeal, intestinal, genitourinary tract and gingival epithelia. For certain medical indications the preparation may be provided in a form suitable for topical application, such as in a cream, spray or droplets.

The formulation according to the invention specifically may be provided in the form of a natural formulation like a dairy product, where the immune complex is provided in the natural context, such as milk, or milk products such as cheese, yoghurt, whey, whey concentrate, or else a synthetic formulation that comprises the isolated or purified substances, e.g. Vitamin A, SIgA, SIgM, SC or immune complexes. Specifically the formulation according to the invention may be provided in the form of a dairy product, a protein bar, a nutritional bar, tablet, capsule, chewing gum, paste, powder, granules, suppository and syrup.

Preferably the formulation is a formulation for oral use.

The formulation according to the invention may particularly be provided for use as a food product and/or for therapeutic use. Specifically the formulation may be provided as a dietary supplement, nutritional management food, food additive or medical food. Specific therapeutic indications are, for instance, infectious diseases, such as of the nasopharynx tract, oropharynx tract, laryngopharynx tract, urogenital tract, eyes and gastric tract, e.g. bacterial overgrowth in the proximal small intestine, recurrent urinary tract infections or chronic bronchopulmonary infections.

A preferred use is the prevention of a disease or disorder caused by a pathogen, including microbial substances or organism, antigens or disease causing agents, such as toxins.

As used herein the term "pathogen" always includes microbial organisms and toxins, also including bacterial, fungal, viral and protozoan cells and products, in particular human or veterinarian pathogens.

The Vitamin A component as used in the preparation according to the invention is specifically obtained from natural sources such as plants or microbes, or enzymatically or chemically synthesized.

The preparation according to the invention may also preferably contain other components such as carbohydrates. The carbohydrates are preferably sourced from whey protein concentrate and are present in the preparation in a concentration between 0 and 95% w/w. More preferably, carbohydrates are present between 30 and 90% w/w. The carbohydrates provide a readily available energy source.

Dextrose is also preferably used as a carbohydrate additive, which may be included in the preparation to assist in preventing agglomeration of the powder and to provide a further form of carbohydrate.

Further preferred additives are whey proteins, which may further stabilise the immunoglobulin preparation, amino acids for nutritional purposes, oligosaccharides, and substances that enhance the physiological value of the preparation.

It may also be preferred to include antimicrobial substances in the formulation according to the invention, such as antibiotics, antivirals, antifungals, antiparasitics, or microbicidal substances, including organic acids, lysozyme, peroxidase, plant essential oils, cations, collodial silver or quaternary ammonium salts.

The preparation according to the invention may be specifically used for treating and/or preventing infections and/or inflammation of the mucosal surfaces, e.g. the gastro-intestinal tract, urogenital tract, respiratory tract, nasal cavity, the eyes or oral cavity, especially after surgery or during hospitalization. For such treatment after surgery at a daily dose of at least 10 mg SIg is preferred.

Doses are typically provided for single or for intermittent use at a specific time interval, e.g. for daily use or in longer intervals (at least every 2, 3, 4, 5 or 6 days, or at least weekly), such as in a retard or slow release formulation. A daily dose of 1 mg to 10 g SIgA and/or SIgM is preferably provided in a formulation for use in humans. A specifically preferred preparation, e.g. for oral use, may contain 500 mg SIg or about 500 mg SIg, e.g. ranging from 400-600 mg Slg.

The preparation according to the invention preferably is used as a food product, e.g. to provide a specific diet to subjects in need thereof, which are e.g. at risk of or suffering from a disease condition caused by a pathogen.

A further preferred use is the prevention of a disease condition caused by a pathogen, including microbial substances or organism, antigens or disease causing agents, such as toxins. For example, hospitalised patients may need to supplement their immune system to reduce the risk of a hospital-acquired infection. Neonatal humans or animals receiving the food supplement according to the invention may have a higher chance of survival in case that they cannot obtain sufficient colostrum from mothers or respective wet nurses. Furthermore, the risk of enteropathogenic disease in animals and humans may be reduced by such food product.

The preparation according to the invention may be used to treat infectious diseases of the mouth, throat, nose and ears, the eyes and esophagus, the gastric and colon tract.

Suitably, the preparation according to the invention may be provided in a formulation, which optionally provides for further nutrients such as proteins, carbohydrates, lipids, and other physiologically active substances.

Exemplary preparations produced according to the invention include dairy milk and whey powders which are a by-product of cheese production. Whey based products according to the invention additionally comprise, for instance, serum albumins, lactalbumin, lactoglobulin, lactoferrin, lactoperoxidase, oligosaccharides, peptides, lactose and minerals, but do not contain vitamin A. In some cases it is preferred to obtain a product from milk or whey from hyperimmunised adults so that the preparation according to the invention contains some increased level of immunoglobulins reactive with a specific group of disease organisms, pathogens or disease related antigens.

The stabilised preparation may have an increased thermostability and/or gastrointestinal stability, as measured by pH stability and/or protease stability of the protein, resulting in an increased recovery or prolonged in vivo half-life. The stabilising effect is particularly important for the mucosal recovery. Upon administration of the preparation according to the invention, the immunoactivity of the SIg may be determined in the mucosa by immunological techniques such as ELISA. An increased level of secretory immunoglobulins in the mucosa indicates an increased recovery.

The SIg recovery is typically measured as mucosal SIg level as compared to a normal value of SIg level, a reference. The normal value of SIg level at a specific site may be the value of the average of a population or the value of the individual or a group of individuals before treatment. Such preparations of the invention are preferred which provide for a (maximal) increase in SIg, an SIg recovery of at least 50%, preferably at least 100 %, more preferably 500%.

According to exemplary embodiments of invention, SIg from human milk is prepared from colostrum, SIg from goat purified from the milk of goats. Vitamin A is admixed to the purified SIg fractions, e.g. beta-carotene or retinylpalmitate and the recovery upon oral administration to human healthy subjects determined by testing samples of saliva or faeces for the respective SIg level. Thereby a synergistic recovery rate can be determined with SIg preparations in combination with the Vitamin A.

According to a further exemplary embodiment, the effect of the preparation according to the invention is tested in an *in vivo* mouse model of SIgA deficiency to survive otherwise lethal bacterial infections, such as with enterotoxic *E.coli.* It can be proven that mice receiving a diet supplemented with preparations according to the invention are effectively protected from a lethal outcome. Specifically the combination of SIgA with retinol may be used to prevent disease conditions following transient IgA deficiency.

According to a further exemplary embodiment, polyclonal SIg in combination with retinyl palmitate is used to prevent the breakdown of oral tolerance (and consequently specific allergic reactions) in aged animals with secretory Ig deficiency.

According to a further exemplary embodiment, monoclonal, polyreactive sIgA antibodies are used in a preparation according to the invention in order to prevent bacterial hypertoxic effects.

According to a specific example, SIg are determined in human subjects before and after treatment of the subject with a preparation containing SIgA, SIgM and vitamin A. Secretory Immunoglobulins in saliva and faeces was determined to be in the range of 8 to 93 mg SIgA per 100 g faeces and 67 to 186 microgram per ml of saliva. The recovery was in the range of 72 to 117%.

According to another specific example, a lateral flow immunoassay for detecting mucosal immunodeficiency is described. Such assay may be employed to identify the subjects in need of SIg treatment, and to allow for personalized treatment.

Yet, according to a further example, secretory immunoglobulin has been prepared from goat milk on the pilot scale with a yield of up to 30 mg per liter.

The foregoing description will be more fully understood with reference to the following examples. Such examples are, however, merely representative of methods of practicing one or more embodiments of the present invention and should not be read as limiting the scope of invention.

### EXAMPLES

### Example 1: Preparation of secretory immunoglobulin from milk

This example describes the pilot scale preparation of secretory immunoglobulin from bovine, sheep and goat milk.

Table1: Process steps for purification of secretory immunoglobulin from milk
1. Delipidation / centrifugation
2. Acid precipitation of casein
3. Removal of precipitate / Centrifugation
4. Depth filtration
5. Ultra-/Diafiltration
6. Preparative Size Exclusion Chromatography (SEC)
7. Analytical SEC, Dot blot analysis
8. SDS-PAGE

### Materials and methods

### Preparation of whey

For all centrifugation steps a Beckman-Coulter™ Avanti J-25 centrifuge with the rotor JLA10.500 was used. Beakers with a nominal volume of 0.5 L were used, but were filled only up to 0.4 L.

### Delipidation

Delipidation was performed by centrifugation at 11827 g (8000 RPM), for 30 minutes at room temperature. The supernatant was used for further preparation. The pellet (fat) was discarded.

### Acidic precipitation of casein

Acidic precipitation was performed by slowly adding a 5% HCl solution under constant vigorous stirring of the delipidated milk until a pH of 4.6 was obtained.

### Removal of precipitate

Removal of the precipitate was performed by centrifugation at 14969 x g (9000 RPM), for 45 minutes at room temperature. The supernatant was used for further preparation. The pellet (precipitate) was discarded.

### Depth filtration

The whey was filtrated using a peristaltic pump (Watson-Marlow X-100) and a sterile filter unit (Sartorius-Stedim Sartobran P 0.45 + 0.2 µm pore size; filtration area 0.1 m²).

### Ultra/Diafiltration

Equipment
Millipore Labscale™ TFF System
Millipore Cogent µScale TFF System
Filtration membranes
GE Healthcare Kvick™ Start 100 kD 50 cm²
GE Healthcare Kvick™ Lab 100 kD 100 cm²

### Concentration and diafiltration of whey

For the Millipore Labscale™ TFF System 3 membranes were used for ultra- and diafiltration of whey, with a total membrane area of 0.015 m². The pump setting was 2 and a transmembrane pressure of approximately 2.5 bar was adjusted. The whey was concentrated ∼ 1:25 and diafiltrated with phosphate buffered saline (PBS) whereas the concentrated whey was buffer exchanged with a 7-fold excess of PBS.

Millipore Cogent µScale TFF System: 3 membranes with a total membrane area of 0.03 m² were used. The pump setting was 40% of the maximum flow, corresponding to approximately 150 mL/min, the transmembrane pressure 2.5 bar.

Permeate flux was measured by weighing permeate sample at distinct time intervals.

### Preparative SEC

### Superose 6

Superose 6 prep grade packed into a HiScale™ column 26/40 was used. The bed height was 32.5 cm and the column volume was 173 mL. The flow rate was 30 cm/h (2.65 ml/min) and the sample volume was 15 mL, corresponding to 4.7% of the column volume (CV). Fractionation was performed from 0.3 CV to 0.57 CV with a fraction size of 5 ml. The equilibration and running buffer was 1 x PBS.

### Superdex 200

A Superdex 200 prep grade HiLoad™ column 26/60 was used. The bed height was 59.7 cm and the column volume was 317 mL. Running conditions were same as described for Superose 6.

### Milk samples

Bovine and sheep milk was obtained form local food stores, all samples of goat whey and goat milk were obtained from Hofkäserei Dörfl (Untere Bergstrasse 1, 3041 Dörfl, Austria).

### Analytical SEC

A HP Chemstation 1100 (Agilent) was used for analytical SEC. Columns were Superose 6 10/300 GL and Superdex 200 10/300 GL, both from GE Healthcare. The flow rate was 0.5 mL/min and the injection volume was 50 µL. The equilibration and running buffer was 1 x PBS. UV signals were recorded at 214 nm.

### SDS-PAGE

Samples were added with DTT to a final concentration of 200 mM and NuPAGE LDS Sample Buffer (4x) and were boiled for 10 minutes. Samples were loaded onto Tris-Acetate 3-8% gels, the running buffer was 1xNuPAGE Tris-Acetate SDS Running Buffer. The running conditions were 150 V, max Ampere, 1.5 hours. The marker was Invitrogen HiMark Pre-Stained High Molecular Weight Protein Standard. Protein staining was performed with Coomassie Blue.

### Dot-Blot

5 µl portions of samples from preparative SEC runs were applied to a nitrocellulose membrane. After air-drying of the samples, membranes were blocked with 3% BSA for 1 hour and washed 3 x with PBS buffer containing 0.1 % Tween 20 (wash buffer). Afterwards membranes were incubated with specific antibody-HRP conjugates for 1 hour. Following conjugates were used: 1) Rabbit-anti-goat-IgM-HRP, product number AAI45P; 2) Rabbit-anti-goat-IgG(H/L)-HRP, product number 5160-2504; 3) Rabbit-anti-goat-IgA-HRP, product number AAI44P (AbD Serotec, Germany). The conjugate stock was diluted 1:30000-1:50000 with wash buffer containing 1% BSA. After washing 3 x with wash buffer, signalling was performed with SuperSignal West Pico Chemiluminescent Substrate (Pierce, Germany) and the Lumi-Imager™ scanner from Boehringer Mannheim.

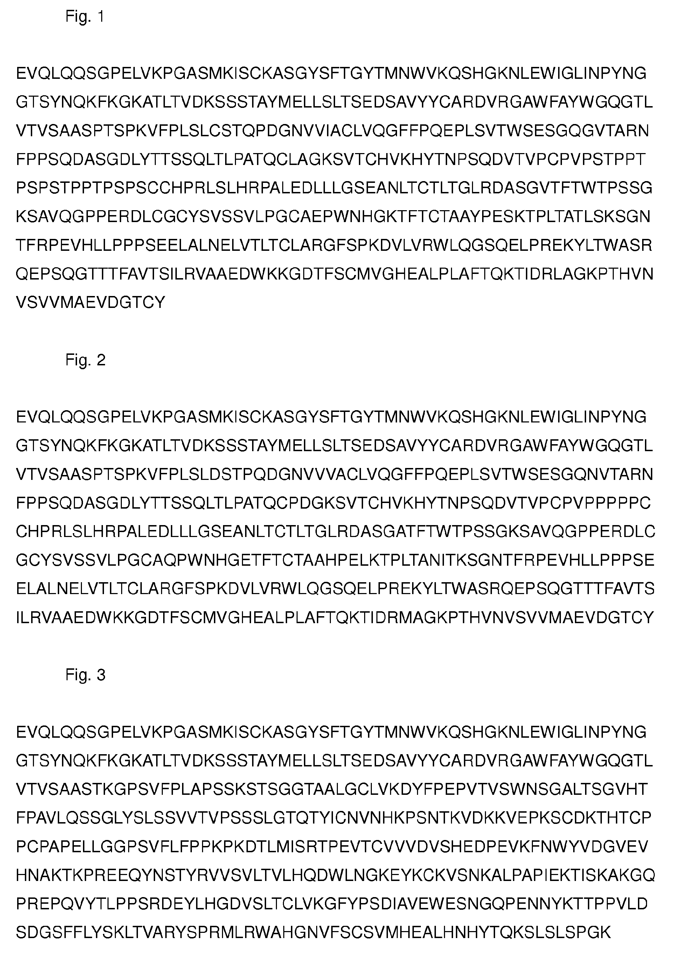

A complete mass balance with regards to SIgA of the process was established and is shown for two goat milk samples (Samples No. 11 and 12) in Table 1. All peak profiles from the HPLC analysis were deconvoluted into its individual immunoglobulins. The SIgA and SIgM content was then calculated based on the purity and the amount of protein obtained from the preparative run data. Fig. 1 shows the SIgA and SIgM yield of various samples from goat milk.

**Table 1: Mass balance, yield and purity of processing of some samples**

| | **Sample 11** | **Sample 5** | **Sample 12** |
|---|---|---|---|
| Initial volume milk [mL] | 900 | - | 900 |
| Volume whey [mL] | 875 | 1000 | 875 |
| Volume concentrated whey [mL] | 35 | 50 | 35 |
| Concentration factor | 1:25 | 1:20 | 1:25 |
| IgA purity | 76% | 61% | 90% |
| Total amount IgA [mg] | 5,7 | 30.8 | 9,8 |

Secretory immunoglobulin from collected frozen human breast milk is prepared according to the same procedure.

A number of different samples are prepared:
Preparation no. 1: 10 mg goat SIgA/SIgM preparation
Preparation no. 2: 10 mg goat SIgA/SIgM preparation plus 100 micrograms retinylpalmitat
Preparation no. 3: 100 micrograms retinylpalmitat (Sigma R1512)
Preparation no. 4: 10 mg human Slg
Preparation no. 2: 10 mg human SIg plus 100 micrograms retinylpalmitat

### Example 2: Recovery of human SIgA in Individuals

This example is to show a surprising synergistic effect between SIg and retinylpalmitate with both, human and goat Slg.

### Testing for serum IgA

Individuals are tested before and after the treatment for IgA in blood by a conventional clinical laboratory. The individuals for the experiments have normal IgA concentrations (> 7 mg IgA / dl serum) in blood before and after the experiment.

### Testing of human SIgA in saliva

Individuals are allowed water ad libitum before, during and after testing with the exception of the 10-min period prior to each saliva collection. The subject is asked to swallow to dry the mouth before the salivette swab (Sarstedt, Nümbrecht, Germany) is placed under the tongue, where it remains for 2 min. The swab is then returned to the salivette tube which is sealed and stored frozen at - 70°C for later analysis. Thawed salivettes are centrifuged at 5000 rpm for 5 min at room temperature.

The concentration of IgA in saliva is determined by a sandwich-type ELISA. For the detection of human IgA, a high protein binding ELISA plate (Nunc Maxisorp) is coated with MT57 antibody (mouse monoclonal anti-human IgA from MabTech, Nacka Strand, Sweden), diluted to 2 microgram per ml PBS, pH 7.4, by adding 100 microliter per well. After overnight incubation at 8°C, the plate is washed twice with PBS (200 microliter per well). The plate is then blocked by adding 200 microliter per well of PBS with 0.05% Tween-20 (TPBS) containing 0.1 % bovine serum albumin (incubation buffer). After an incubation for 1 hour at room temperature the plate is washed five times with TPBS.

A human IgA standard (MabTech, Sweden) is prepared by reconstituting the lyophilized standard in 500 microliter PBS to make a stock solution of 50 micrograms per milliliter. The stock solution is used immediately or stored frozen at -20°C for future use. For the assay, dilutions are prepared using the standard range as guideline (0.2 to 100 ng IgA per ml) according to the manufacturer. Both, samples as well as standard are diluted in incubation buffer. Samples are assayed at dilutions of 1 to 1,000 and and 1:5,000.

100 microliters per well of samples or standard dilutions are incubated for 2 hours at room temperature.

After five times washing with TPBS, 100 microliter per well of Monoclonal Antibody to Human IgA (Secretory component) - HRP conjugate (Acris Antibodies GmbH, Germany, cat. No. AM20261 HR-N), diluted 1 to 1,000 in incubation buffer, is added. The plate is incubated for 1 hour at room temperature. The plate is the washed five times with TPBS. After washing, 100 µL/well of tetramethylbenzidine (71.7 µg/mL) and 0.05% H₂O₂ are added to the wells and incubated for 10 min at room temperature (21 °C). The reaction is stopped with 50 µL/well of 2.5 N of H₂SO₄. Extinctions are measured with a microplate spectrophotometer at a wavelength of 450 nm. Sample concentrations are calculated by comparison within the linear range of the standard curve.

### Treatment

The treatment schedule is two times per day for 3 weeks by oral application of 5 ml (50 mg) of the respective preparation.

SIgA recovery in saliva is tested before treatment as well as one day after end of treatment.

**Table 2 Recovery after treatment with various Slg-preparations**

| | before treatment | | treatment | after treatment | | |
|---|---|---|---|---|---|---|
| Individual | serum IgA (g/L) | saliva SIgA (mg/L) | | serum IgA (g/L) | saliva SIgA (mg/L) | SIgA % recovery* |
| A | 2,47 | 150 | hSIgA | 2,22 | 186 | 124 |
| B | 1,93 | 143 | hSIgA + retinylpalmitate | 2,51 | 306 | 214 |
| C | 2,2 | 113 | retinylpalmitate | 1,85 | 96 | 85 |
| D | 1,89 | 254 | cSIgA | 2,12 | 213 | 84 |
| E | 2,51 | 126 | cSIgA + retinylpalmitate | 2,43 | 236 | 187 |

| | | | | | | |
|---|---|---|---|---|---|---|
| * ... reference (before treatment) = 100% hSIgA ... human SIgA cSIgA ... caprine SIgA | | | | | | |

Results summarized in table 2 suggest that recovery of SIgA in saliva is highest with a combination of Slg and retinylpalmitate.

### Example 3: Prophylactic treatment of mice with various SIgA combinations to prevent lethal bacterial infections after induction of IgA deficiency

The example is able to show a synergistic effect of a combination of SIgA and a vitamin A supplement in preventing of health complications which may follow SIgA deficiency.

For this purpose, mice are treated with cyclophosphamide to induce a transient mucosal IgA deficiency. During this period of immune deficiency animals are infected with an otherwise non-lethal dose of enteropathogenic *E. coli.*

Mice are being treated during, before and after immune deficiency with various substances known to have an influence on the immune system.

2-5 week old Balb/c mice, female are fed either a basic diet alone (control group) or in addition to the basic diet one of the supplements (20 mice per group).
Supplement 1: beta-carotene
Supplement 2: human SIgA (Sigma I1010)
Supplement 3: human SIgA plus beta-carotene

The basic diet is based on wheat flour and contains additionally (per kg of feed) 150 g cellulose, 50 g lipid, 10g linoleic acid, 250 g casein, 5 g calcium, 0.5 g chloride, 0.5 g magnesium, 0.5 g sodium, 3 g phosphorus, 2 g potassium, 8 mg copper, 35 mg iron, 10 mg manganese, 10 mg zinc, 150 microgram iodine, 150 microgramm molybdenum, 150 microgram selenium, 300 microgram retinol, 25 microgram cholecalciferol, 22 mg alpha-tocopherol, 1 mg phylloquinone, 0.2 mg d-biotin, 2 g choline bitartrate, 0.5 mg folic acid, 15 mg niacin (nicotinic acid), 15 mg Capanthotenate, 7 mg riboflavin, 8 mg pyridoxin-HCL, 10 microgram vitamin B12 and 5 mg thiamin-HCl.

200 microgram alpha-tocopherol, 100 mg L-ascorbic acid, 70 mg Vitamin B2, 80 mg Vitamin B6, 1 microgram Vitamin B12, 2 microgram selen (as selenite) and 100 microgram zinc per daily dose.

Beta-carotene supplement is 50 microgram beta carotene per day per animal.

Human SIgA supplement is a daily dose of 10 mg human SIgA per animal.

Feeding (with basic feed or basic feed plus supplements) is started at day 0.

After 10 days, 10 mice of each group are treated with a single i.p. dose of cyclophosphamide (200 mg/kg body weight). After additional 5 days, mice are challenged with enterotoxic E. coli. For infection, the mice are orally inoculated with a single gavage of 10,000 CFU of E. coli O157:H7 strain. Survival rate of mice is recorded. Supplements with hSIgA in combination with beta-carotene are protecting mice best from the infection.

### Example 4: Monoclonal, polyspecific SIgA in combination with vitamin A to prevent fatal infections in aged animals with secretory IgA deficiency

This example is able to show a synergistic effect of polyspecific monoclonal SIgA and a vitamin A supplement in a mouse model with age related secretory IgA deficiency and the superiority of SIgA compared to IgG.

The variable regions of the polyreactive antibody 2E4 (Cell Host Microbe 2007; volume 1 , pages 51-61) are cloned to be expressed as dimeric IgA in animal cells. SIgA is then produced by mixing purified dimeric IgA and purified secretory component with subsequent purification of the reconstituted complex.

The antibody, together with vitamin A is fed to aging BWF1 mice which have a marked decrease in IgA levels in feces. Mice are then challenged orally with E. coli and the symptoms as well as the death rates are recorded. As controls, young BWF1 mice with normal levels of IgA in feces and 2E4-IgG as well as vitamin A alone and monoclonal SIgA alone are used.

The vector pIRES/neo (Clontech) is used to clone the kappa light chain of 2E4 together with the J-chain to result in pIRES-kappa2E4J.

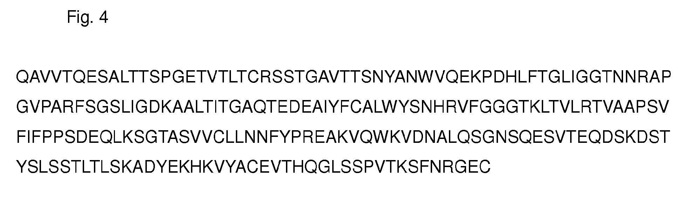

The protein sequence of the kappa light chain of 2E4 is to be seen in figure 4.

This sequence, provided with an immunoglobulin leader peptide at the N-terminus is back-translated into DNA with codons optimized for CHO expression. For construction of the J-chain polypeptide, the sequence ref. P01591 (IGJ_HUMAN), UniProtKB/Swiss-Prot, is used. This sequence is backtranslated into nucleic acid with optimal codon usage for expression in CHO.

The heavy chain of the respective construct (for 2E4 IgA1 the protein sequence 2E4 Calpha1 - see figure 1 - is used; for 2E4 IgA2 the protein sequence 2E4 Calpha2, see figure 2, is used; for 2E4 IgG the protein sequence Cgamma1, Figure 3, is used) is provided with a secretory signal peptide (immunoglobulin leader) and back-translated into DNA to be synthesized and cloned in a mammalian expression vector (pCDNA3.1)

The light chain (and J-chain) coding plasmid pIRES-kappa2E4J is cotransfected with one of the three heavy chain encoding plasmids respectively in CHO cells.

CHO cells are being transfected in a standard transfection procedure with mixtures of plasmids: Linearized pIRES-kappa2E4J (coding for 2E4 kappa chain and the J-chain) is combined with linearized plasmids encoding for 2E4 heavy chain Calpha1 and 2E4 heavy chain Calpha2 (based on pCDNA3.1), respectively. The transfection is followed by a standard selection procedure to yield stable clones (G418 concentration may be increased to 1000 micrograms per ml selection medium).

The cells are incubated for 2-3 weeks before the supernatant fluids are harvested and screened for immunoglobulin production by enzyme-linked immunosorbent assay (ELISA).

For ELISA, microplates are coated with anti-J chain antibody (goat anti-J chain, Santa Cruz, sc-34654). After washing, cell culture supernatants (diluted 1:2 and 1:10) are added. Subsequently to incubation and washing, anti human-IgA alpha chain-HRP conjugate is used for detection of cell lines expressing IgA.

Purification of recombinant dIgA from culture supernatant of selected clones is performed by affinity chromatography utilizing an anti-human-IgA coupled sepharose (Sigma A2691) according to the manufacturer and standard affinity chromatography procedures.

Purification of 2E4-IgG is performed by Protein A affinity chromatography of culture supernatants of the respective transfectants.

### Expression of recombinant human secretory component in mammalian cells

This part of the example describes the establishment of mammalian cells expressing Secretory Component.

The Secretory Component Protein sequence of NCBI ACCESSION 2OCW_A is used for design of the vector. The signal peptide sequence of NCBI ACCESSION AAB20203 is added at the N-teminus.

The protein Sequence is reverse translated into DNA (optimized for mammalian cell expression) and synthesized de novo (Geneart, Germany).

The gene is inserted at into the mammalian protein expression vector pCDNA3.1+ (invitrogen, USA).

To generate stable transfectants, plasmids are linearized with Pvul and subsequently transfected into CHO-K1 cells using Lipofectamine 2000 according to the manufacturer (Invitrogen). Twenty-four hours following transfection, cells in each T-flask are split into five 100-mm petri dishes and incubated in the selection medium. The concentration of G418 is 200-400 microgram/mL. The selection medium is changed every third day. Drug resistant clones can be seen after approximately 2 weeks, identified under the microscope, and handpicked using a pipetman. Selected colonies are cultured in 96-well plates in the presence of G418 for 2 weeks. Growing cells are split into duplicate wells and supernatants are tested for expression of Secretory Component in a standard ELISA. In short, goat anti-Secretory Component antibody (Acris Antibodies AP21476FC-N) is coated to the plates, after incubation and washing, supernatants of the cells are added (1:2 and 1:10 diluted). After incubation and washing, mouse anti-human-SC is added (Sigma I6635) and subsequently detected with anti-mouse-IgG-HRP. Selected positive clones are used for purification of Secretory Component.

Secretory component can be purified from supernatants of cell culture of selected positive clones by combining successive chromatographic steps involving Q-Sepharose and Superdex 200. Column fractions containing recombinant SC are identifed by Western blot.

Purified dimeric IgA is reconstituted to SIgA with recombinant Secretory Component from CHO cells.

Reconstitution of dIgA-Secretory Component Complexes is performed in vitro by mixing equimolarily purified dIgA and purified Secretory Component (fucosylated and non-fucosylated respectively) overnight in PBS buffer at a concentration of 10 micrograms per 100 microliters.

Reconstituted complexes are loaded onto a non-reducing and a reducing 6% SDS-polyacrylamide gel respectively, blotted to polyvinylidine difluoride membrane, and detected with antiserum against secretory component.

Covalent reconstitution takes place as indicated by the shift of the secretory component to the position of dIgA and pIgA molecules. Under reducing conditions, only free Secretory Component can be detected to a similar extent in every lane, indicating that Secretory Component and IgA are linked by disulfide bridges.

The complexes are further purified by preparative HPLC.

BWF1 mice are maintained under specific pathogen-free condition. Female mice age 7-10 weeks are used as young mice, whereas 8- to 10-month old mice with IgA levels in feces below 50 micrograms/mL are used as aged SIgA-deficient mice.

### Detection of fecal IgA

Fecal pellets (100 mg) are placed into 1.5 ml of microcentrifuge tubes, 1 ml (10 w/v) of PBS added, and incubated at room temperature for 15 min. Samples are vortexed, left to settle for 15 min, revortexed until all material is suspended, then centrifuged at 12,000 rpm for 10 min. The supernatant is removed and stored at -80°C or immediately tested on ELISA for IgA using a mouse IgA ELISA Quantitation kit (Bethyl Laboratories). Microtiter plates are coated with goat anti-mouse IgA affinity purified Ab and incubated for 60 min. Plates are washed with PBS containing 0.05% Tween 20 (PBST) and each well is blocked with 200 microliters of 50 mM Tris, 0.15 M NaCl, and 1% BSA (pH 8.0) for 30 min. Plates are washed with PBST, 100 microliters of test samples and standards are added per well, and incubated for 60 min. Plates are washed with PBST and 100 microliters of HRP-labeled goat anti-mouse IgA Fc-specific Abs is added to each well and incubated for 60 min. Plates are washed, developed for 30 min with HRP substrate (3,3,5,5-tetramethylbenzidine), stopped with H2SO4, and read at 450 nm with a microplate reader.

### Treatment of mice

treatment groups:
treatment 1: beta-carotene alone (daily dose: 300 microgram beta-carotene)
treatment 2: polyspecific monoclonal 2E4 SIgA (daily dose: 10 mg)
treatment 3: polyspecific monoclonal 2E4 SIgA plus beta-carotene
treatment 4: polyspecific monoclonal 2E4 IgG (daily dose: 20 mg) plus beta-carotene
mouse-groups:
group A: young mice
group B: aged mice with SIgA-deficiency

Mice of group A are treated with treatments 1 and 2, mice of group B are treated with treatment 1, 2, 3 and 4 (at least 10 mice per treatment group). Treatment is performed daily for 21 days, infection of mice with bacteria is started on day 10 after start of treatments.

### Infection of mice

Escherichia coli B41 (O101 :K-, K99, F41) can be obtained from the American Type Culture Collection (no. 31619). E. coli B41 is inoculated on a Trypticase Soy Agar (BD Biosciences) plate, which is then incubated for 24 h at 37°C. The bacteria are washed off this plate with 5 ml of sterile PBS. A suspension of E. coli B41 is prepared in this way and sterile DMSO is added to a final concentration of 10% v/v. The culture is dispensed aseptically in 500 microliters of portions and rapidly frozen in liquid nitrogen. The viable count of all E. coli suspensions is measured at the time of inoculation. Bacterial suspensions are recovered from frozen storage and resuspended to comprise 10⁹ CFU in 100 microliters of PBS. Each mouse is given 100 microliters of bacterial suspensions directly into the stomach using a ball-tipped gastroesophageal needle for 3 consecutive days. Infected mice are observed daily and mortality is recorded. Mortality in aged mice is lowest in treatment group 3 (SIgA plus beta carotene).

### Example 5: Polyclonal SIgA in combination with vitamin A to prevent allergoid reactions as a consequence of breakdown of oral tolerance in aged animals with secretory IgA deficiency

The experimental example is able to show a synergistic effect of polyclonal SIgA in combination with vitamin A and the possibility to protect animals with SIgA-deficiency from breakdown of oral tolerance due to mucosal IgA deficiency.

It has been described that aged BWF1 mice show a breakdown of the mucosal immunity (J Immunol. 2005; volume 174 pages 5499 ff.). The resulting breakdown of oral tolerance allows to trigger an allergic reaction.

Mice with mucosal IgA-deficeny (as measured by fecal IgA concentration, see previous example) are treated with a combination of SIgA and vitamin A. As controls, either SIgA alone or vitamin A alone is being used. After a certain treatment period, mice are fed ovalbumin. After a period of time, mice are immunized s.c. with ovalbumin in complete Freund's adjuvant.

Tolerance to this immunization is measured by detection of levels of ovalbumin-specific IgG antibodies in serum.

BWF1 mice are maintained under specific pathogen-free condition and are fed with a standard diet containing all nutrients, vitamins and minerals. 8- to 10-month-old mice with moderate to severe proteinuria (300 to 1000 mg/dl, Albustix; Bayer) are used as aged mice. SIgA-deficiency is checked as described in the previous example.

Mice are treated daily for 2 weeks either with 10 mg goat SIgA alone, with 100 micrograms of retinylpalmitate alone or a combination of SIgA and retinylpalmitate. The treatment is continued during the following challenging experiment:
Induction of systemic unresponsiveness to ovalbumine (OVA, Sigma-Aldrich) is performed as described in J. Immunol. 2001; volume 166, pages 2055ff.). Briefly, mice are given 25 mg of OVA in 250 microliters of PBS by gastric intubation on day 0. Control mice receive PBS only. On days 7 and 21, mice are immunized and challenged s.c. with 100 micrograms of OVA in 100 microliters of complete Freund adjuvans (CFA, Difco).

OVA-specific Ab in the serum is measured 7 days after the second s.c. Immunization. ELISA plates (Corning Life Sciences) are coated overnight at 4°C with 1 mg/ml OVA in PBS. Blocking is done with 200 microliters of 1% BSA in PBS for 1 h at 37°C. Serial dilutions of serum in 1% BSA/PBS are prepared and 100 microliters are added per well in duplicate. Following incubation at 37°C for 4 h, HRP-labeled goat anti-mouse IgG Fc-specific Abs (Bethyl Laboratories) are added and incubated overnight at 4°C. Color is developed with 1.1 mM ABTS (Sigma-Aldrich) in 0.1 M citrate-phosphate buffer (pH 4.2) containing 0.01% H₂O₂. Mice treated with secretory immunoglobulin supplemented with retinylpalmitate show a lower titer of OVA specific antibodies in serum compared to the other treatment groups.

### Example 6: Secretory Immunoglobulins in saliva and faeces before and after treatment of an individual with a preparation containing SIg and beta-carotene

In order to show the advantageous effects of a combined treatment of subjects with beta-carotene and secretory immunoglobulin, an untrained individual with confirmed normal IgA levels in blood is exposed to extreme physical stress for 3 days (mountain climbing at an altitude of about 3000 m above sea level in the alps for 8 hours per day). The levels of SIgA in saliva and faeces are measured 3 days before the mountain climbing tour and afterwards (1 and 6 days afterwards).

The individual receives from day -2 until day 6 after the exercise daily supplementation of secretory immunoglobulin isolated from goat whey (daily dose 100 mg Slg) plus 25 mg beta carotene (Carotaben, Almirall Hermal GmbH, Austria) in addition to normal food. In control experiments, the individual receives beta-carotene alone and secretory immunoglobulin preparation from goat whey alone, respectively. In both experiments with supplementation with beta-carotene, the beta carotene supplementation starts 2 weeks before the exercise.

### Testing of Serum IgA

Individuals are tested for IgA in blood by a conventional clinical laboratory. The individuals for the experiments have normal IgA concentrations (> 7 mg IgA / dl serum) in blood before and after the experiment.

### Testing of human SIgA and caprine SIgA in saliva

Individuals are allowed water ad libitum before, during and after exercise with the exception of the 10-min period prior to each saliva collection. The subject is asked to swallow to dry the mouth before the salivette swab (Sarstedt, Numbrecht, Germany) is placed under the tongue, where it remains for 2 min. The swab is then returned to the salivette tube which is sealed and stored frozen at - 70°C for later analysis. Thawed salivettes are centrifuged at 5000 rpm for 5 min at room temperature.

The concentration of IgA in saliva is determined by a sandwich-type ELISA. For the detection of human IgA, a specific pair of monoclonal antibodies from MabTech (Nacka Strand, Sweden, Product no. 3860-1AD-6)) are used:
A high protein binding ELISA plate (Nunc Maxisorp) is coated with MT57 antibody (anti-human IgA), diluted to 2 microgram per ml PBS, pH 7.4, by adding 100 microliter per well. After overnight incubation at 8°C, the plate is washed twice with PBS (200 microliter per well). The plate is then blocked by adding 200 microliter per well of PBS with 0.05% Tween-20 (TPBS) containing 0.1 % bovine serum albumin (incubation buffer). After an incubation for 1 hour at room temperature the plate is washed five times with TPBS.

A human IgA standard is prepared by reconstituting the lyophilized standard in 500 microliter PBS to make a stock solution of 50 micrograms per milliliter. The stock solution is used immediately or stored frozen at -20°C for future use. For the assay, dilutions are prepared using the standard range as guideline (0.2 to 100 ng IgA per ml) according to the manufaturer. Both, samples as well as standard are diluted in incubation buffer. Samples are assayed at dilutions of 1 to 1,000 and and 1:5,000.

100 microliters per well of samples or standard dilutions are incubated for 2 hours at room temperature.

After five times washing with TPBS, 100 microliter per well of MT20 (anti-human IgA)-alkaline phosphatase conjugate, diluted 1 to 1,000 in incubation buffer, is added. The plate is incubated for 1 hour at room temperature. The plate is the washed five times with TPBS. 100 microliter per well of chromogenic substrate (1 mg p-nitrophenylphosphate per ml of 1 M diethanolamine buffer pH 9.8).

The plate is incubated at room temperature for 30 minutes or until sufficient color develops. To stop the reaction, 50µL of 2N NaOH are added to each well. Absorbance at 405nm is measured. Sample concentrations are calculated by comparison within the linear range of the standard curve.

Testing for goat IgA is performed by the same procedure as for the human IgA described above, the differences being the specific antibodies used:
For coating, rabbit antibody to goat IgA (ab112863 from Abcam, UK) is diluted 1 to 100 in sodium cabonate buffer, pH 9.7.

The detection antibody is rabbit anti-goat IgA-Alkaline phosphatase conjugate, (abcam no. ab112864, Abcam, UK) diluted 1:1,000 in incubation buffer.

SIgA in saliva is expressed in microgram IgA per ml of saliva

### Testing of human SIgA and caprine SIgA in faeces

Fresh stool samples are collected, aliquotted and stored at -80°C until tested. Each frozen faecal sample is weighed and then rapidly thawed while it is resuspended (for each gram, 2 milliliters) and homogenized under agitation in phosphate buffered saline, pH 7.4 with 2 nM phenyl-methyl-sulfonyl-fluoride as protease inhibitor, and 1 mM sodium azide as bacteriostatic agent. Each specimen is immediately submitted to a first centrifugation at 1,200 g for 15 min at 4°C, followed by a further centrifugation at 8,000 g for 30 min at 4°C. The clear supernatant is collected, distributed in 1.5-mL aliquots, and stored at -80°C.

The IgA concentration is tested by ELISA as described above. IgA concentrations are referenced to the original weight of the sample and are expressed in micrograms per 100 g faeces sample.

The data in table 3 below show that the combination of beta-carotene with secretory immunoglobulin allows for a higher recovery of human sIgA in saliva and faeces as compared to either beta-carotene supplementation alone or secretory immunoglobulin preparation alone.

**Table 3: SIgA levels and recovery of human IgA in faeces and saliva before and after exercise at different treatments**

| | 3 days before exercise | | day1 after exercise | | day 6 after exercise | | Recovery* in saliva (%) | Recovery* in faeces (%) |
|---|---|---|---|---|---|---|---|---|
| Treatment | faeces | saliva | faeces | saliva | faeces | saliva | | |
| Beta-carotene | 88 | 128 | 8 | 67 | 11 | 98 | 77 | 13 |
| Beta-carotene plus caprine SIgA/SIgM | 65 | 113 | 14 | 123 | 47 | 132 | 117 | 72 |
| caprine SIgA/SIgM | 93 | 186 | 12 | 84 | 34 | 104 | 56 | 37 |

| | | | | | | | | |
|---|---|---|---|---|---|---|---|---|
| *... Reference (before exercise) = 100% | | | | | | | | |

### Example 7: Lateral Flow Immunoassay for determination of mucosal immunoglobulin deficiency

A simple and rapid lateral flow immunoassay can be used to decide on the mucosal immunostatus regarding SIgA and SIgM of an individual.

Such an on-site assay can be optimized to show positivity at a certain treshold with samples from mucosal sites such as e.g. saliva, feces extract, tears and sweat.

Lateral flow tests (Lateral Flow Immunochromatographic Assays) are a simple devices intended to detect the presence of a target analyte in a sample. Often produced in a dipstick format, lateral flow tests are a form of immunoassay in which the test sample flows along a solid substrate via capillary action. The sample encounters a coloured reagent which mixes with the sample and transits the substrate encountering lines or zones which have been immobilized. Depending upon the analytes present in the sample the coloured reagent can become bound at the test line or zone. A scheme of such an assay to detect secretory IgA in a mucosal sample is shown in Figure 2.

A lateral flow immunoassay can be performed within 15 min and can be used to even by non-trained individuals to detect potential mucosal immunodeficiencies.

In this example the setup of an assay for human secretory IgA in saliva is described. For the optimization of such an assay, purified human SIgA and, as a matrix, SIgA-negative saliva from a person with IgA deficiency is used.

Such assays with certain specifications can be developed with contracting companies such as Kestrel Biosciences (Carlsbad, USA), Biocare Diagnostics (Xiangzhou, China), Biotem (France) and others.

Alternatively, the assay is designed by using a generic Rapid Assay Device (gRAD, RapidAssays, Copenhagen, Denmark). Basically, a biotinylated capture reagent, a gold-labelled detection reagent and the sample is mixed and then applied to a lateral flow assay device. The gRAD device used in this example contains an immobilized biotin-binding zone (zone A) and an immobilized anti-mouse antibody zone (control zone, zone B).

The capture reagent in this example is a biotinylated sheep anti-human-Secretory Component-polyclonal antibody.

The gold labelled detection antibody in this example is a mouse anti-human-IgA monoclonal antibody.

The detection and capture reagents are produced according to the instructions provided by RapidAssays, Denmark. The assay is optimized according to the instructions of the RapidAssays reagent kits.

### Biotinylation:

sheep anti-human-Secretory Component-polyclonal purified antibody (Biorbyt Ltd., UK, prod.no. orb20680) is first mixed with biotinylation reagent which reacts with free primary amine present on the protein. The non-protein coupled biotin reagent is then removed via chromatography. For use with the gRAD the linker should be as long as possible. EZ-Link NHS-PEG12-Biotin, Biotin-NHS, Biotin-LC-NHS and Biotin-PEG4-NHS (Pierce) should be tried and compared in the Assay optimization phase.

PBS pH 7.4 and 100mM carbonate buffer pH8.0, respectively are used for the biotinylation of the sheep anti-human-Secretory Component antibody (it must not be in Tris buffer or contain sodium azide as these will block conjugation). The antibody concentration should be at 1 mg/ml.

2µl of biotinylation stock (Stock concentrations in mg/ml DMSO: Biotin-NHS (40), Biotin-LC-NHS (53), Biotin-PEG4-NHS (69), Biotin-PEG12-NHS (110)) is added per mg of antibody.

The reaction mixture is then mixed at room temperature in the dark for 2 hours. The remaining active biotin-NHS is blocked with the addition of 10 µl of 3 M ethanolamine and incubating for a further 30 minutes.

Gel filtration / buffer exchange with Sphadex G25 media is used to remove the free biotin. For smaller volumes gel filtration can be carried out with small disposable columns for example a PD10 (GE Healthcare). The process is repeated to remove as much of the free biotin as possible as it will compete for binding to the test line lowering the resulting response.

### Preparation of the colloidal gold coated detection reagent (according to RapidAssays instructions):

Monoclonal mouse anti-human IgA antibody (Merck/Millipore Calbiochem no. 411420 Mouse Anti-Human IgA, Fab Fragment Specific (HP6123)) is used and is prepared to be at a concentration of 1 mg/ml or greater and should be in a 0.5 X PBS buffer solution.

Naked Gold Sol 40 nm and Naked Gold Sol 20 nm are used.
1. Shake or swirl gold to resuspend any settled gold. Place 0.5 ml Naked Gold sol into ten (10) clean individual test tubes.
2. Label each tube with the pH value (or, 1 through 10) from the provided pH charts: pH5.4, pH6.6, pH7.3, pH7.8, pH8.2, pH8.4, pH8.8, pH9.2, pH9.6, pH10.1
3. Use the pH charts to add varying amounts of buffer in microliters to each test tube. Shake to mix.
pH Charts for Optimal coating at a pH of 5-11 (per 0.5 ml of gold)

| **Tube #** | **pH** | **Buffer A** | **Buffer B** |
|---|---|---|---|
| 1 | 5.4 | 9 µl | 1 µl |
| 2 | 6.6 | 8 µl | 2 µl |
| 3 | 7.3 | 6 µl | 4 µl |
| 4 | 7.8 | 4 µl | 6 µl |
| 5 | 8.2 | 2 µl | 8 µl |

| **Tube #** | **pH** | **Buffer C** | **Buffer D** |
|---|---|---|---|
| 6 | 8.4 | 10 µl | 0 µl |
| 7 | 8.8 | 8 µl | 2 µl |
| 8 | 9.2 | 6 µl | 4 µl |
| 9 | 9.6 | 4 µl | 6 µl |
| 10 | 10.1 | 2 µl | 8 µl |

4. Place each tube on a low speed vortexer and add antibody solution (See Sample Preparation Section). Mix thoroughly (about 2 to 3 seconds).
Ideally, for the 40 nm gold, 7 µl of a 2 mg/ml solution of antibody is optimal. For the 20 nm gold, ideally, 14 ul of a 2 mg/ml solution of antibody is optimal.
5. A deepening purple colour and/or black precipitate on some tubes indicates that the antibody is below its iso-electric point, leading to cross-linking of individual gold sols. Cross-linked sols cannot be used in immunological assays and should be discarded. Deep purple sols are usually mostly inactive as well. Only tubes with a slight purple colour or no change in colour are useful for immunological assays.
6. Allow the reaction to continue for a total of 30 minutes.
7. Stop the reaction by the addition of 50 µl of Blocking Stabilizer Solution.

It is best to allow the blocker to react for an additional 16 hours at room temperature.

In order to test the effectiveness of the conjugation reaction, 10 µl of coated gold sol (prior to the addition of the BSA blocking solution) is mixed with 10 µl of 1 M NaCl. Sols with incomplete coating will fall out of solution (turn black), while completely coated sols will remain stable (red).

The Lateral Flow assay is then optimized with the reagents as described above and gRAD devices according to the manufacturer (RapidAssays, Denmark). Human SIgA from colostrum (Fitzgerald, US, cat. no. 31-AI03S), is used for optimization procedures. As matrix for spiking experiments saliva from a IgA deficient person is used (negativity for IgA in saliva is being tested by a standard procedure such as ELISA or nepheliometry). As a positive control, human SIgA mixed with IgA-negative saliva is used (concentrations of 5 and 9 and 10 and 20 microgram SIgA/dL saliva are used). Positive testing in the gRAD device is set to 10 mg SIgA/ dL, i.e. a saliva sample containing 10 or more mg SIgA /dL will show a signal in zone A and in control zone B, whereas a saliva sample containing less than 10 mg SIgA / dL will show a signal only in the control zone (zone B). The optimization for the threshold is being done by varying the concentrations of antibodies, the ratio between the biotinylated and gold-labeled antibodies and the volumes used in relation to the saliva sample.

The assay can be optimized to other thresholds, other matrices (such as faeces extract, tears, sweat) and for other immunoglobulins (e.g. SIgM, combined SIgA/SIgM, non-human secretory immunoglobulins) accordingly. To allow for a convenient readout, a comparatory signal can be provided on the strip, on the device, on a leaflet or on the package which may even allow for a semi-quantitative readout.

### Example 8: Protection of newly born piglets from severe signs of Clostridium difficile associated disease

This example is based on the *Clostridium difficile* infection model described in Steele et al. (2010) The Journal of Infectious Diseases, volume 201, pages 428-34. The example shows that it is possible to protect animals which are deficient in secretory immunoglobulins. During the first postnatal period mucosal immunity is clearly deficient in that only traces of SIgA and SIgM occur in external secretions, whereas some IgG is often present as a result of 'leakage' from the mucosal lamina propria, which contains readily detectable maternal IgG because of placental transfer.

The first set of experiment compares treatment with a secretory immunoglobulin preparation enriched with retinylpalmitate versus treatment with milk replacer containing Vitamin A:

### Experimental details

Seven gnotobiotic piglets were delivered via Cesarean section and transferred to sterile isolators for the duration of the study. Beginning at age of 3 days, the piglets were divided into two groups: 4 piglets received Similac (Abbott) and Whey Protein Isolate (WPI) from bovine milk (containing 700 mg non-denatured secretory immunoglobulins / 30 g WPI, enriched with 800 µg retinylpalmitate / 30 g WPI) as a preventative, and 3 piglets received only Similac to serve as controls. Piglets in the first group received 10 g whey powder suspended in Similac milk replacer at feedings 3 times daily (total of 30 g per day). At 5 days of age, all piglets were orally inoculated with 10⁶ spores of *C. difficile* strain UK6. Treatment with whey powder continued for one week, and piglets were euthanized earlier if they showed severe signs of disease such as lethargy, anorexia, wasting, dehydration, or respiratory distress. At the end of the study each piglet received a necropsy to evaluate the extent of gastrointestinal and systemic lesions.

### Experimental Results:

### Secretory Immunoglobulins Group: (piglets #3,4,7,8)

All four piglets consumed the whey protein isolate containing the secretory immunoglobulins suspended in the Similac milk replacer well. All 4 piglets in this group developed mild, yellow, pasty to mucoid diarrhea 4 days post-inoculation which continued for the remainder of the study. Piglets 4, 7, and 8 also developed mild anal inflammation 4 days post-inoculation. Piglets 3 and 4 continued to have normal appetites and weight gain until the study endpoint after 1 week of treatment, and did not display any systemic clinical signs of illness. Piglets 7 and 8 had normal appetites, weight gain and activity until 6 days post inoculation, at which point, both piglets developed systemic disease including sudden onset of lethargy, anorexia, and respiratory distress and died or were euthanized.

Necropsy examination of all piglets revealed that piglets 7 and 8 had severe large intestinal lesions including dilatation, hyperemia, mesocolonic edema, and pseudomembrane formation. Additionally, both of these piglets had abdominal and pleural effusions, which likely accounted for the sudden deterioration of these piglets, despite having normal appetites. Piglets 3 and 4 had moderate large intestinal lesions with mesocolonic edema, dilatation, and hyperemia, without pseudomembrane formation. No systemic lesions were noted in these two piglets.

### Untreated, Infected Control Group: (piglets 2,5,6)

Pig 2 developed yellow pasty-watery diarrhea 4 days post-inoculation, and pigs 5 and 6 developed brown-yellow, pasty diarrhea 1 day post inoculation which continued for the duration of the study. All 3 piglets in this group began to display signs of severe systemic illness between 5-6 days post-inoculation and were euthanized.

On necropsy examination, all 3 piglets in this group had severe large intestinal lesions including dilatation, hyperemia, mesocolonic edema, and pseudomembrane formation. All piglets had systemic lesions of abdominal and pleural effusion and cranial-ventral lung consolidation.

### Overall Clinical Impression:

While all of the control group piglets developed severe *C. difficile* infection and were euthanized due to severe clinical signs, 2/4 piglets treated with the secretory immunoglobulin preparation survived, with no severe signs of illness. Additionally, all 4 of the whey protein isolate treated piglets maintained superior body condition to the control piglets.

The second set of experiment describes a comparison between piglets treated with a secretory immunoglobulin preparation supplemented with Vitamin A (whey protein isolate enriched with retinylpalmitate) and piglets treated with a secretory immunoglobulin preparation (whey protein isolate).

### Experimental Methods:

Four piglets were delivered via cesarian section and transferred to one sterile isolator for the study. All piglets were evenly sized, and being from a small litter, were slightly larger than average. At 3 days of age, the piglets were divided into two groups: piglets 1 and 2 receiving whey powder containing non-denatured secretory immunoglobulins supplemented with retinylpalmitate and piglets 3 and 4 receiving whey powder containing non-denatured secretory immunoglobulins. All piglets were given 5 g of the designated whey powder twice daily by suspending in 300-400 ml of Similac milk replacer during the morning and evening feedings. At 5 days of age, all piglets were inoculated with 10⁶ spores of *C. difficile* strain UK6 via oral gavage. Piglets continued to receive whey powder twice daily after dosing, for up to 7 days.

### Experimental Results:

### Secretory immunoglobulin plus retinylpalmitate group: (piglets #1 and #2)

Both piglets consumed the whey powder in the milk and had normal appetites throughout the study. Both piglets developed mild diarrhea 2 days after inoculation. The mild, pasty to watery, yellow diarrhea continued to the experimental endpoint, 7 days after inoculation. Neither piglet in the group displayed any severe clinical signs of CDI and had a normal body condition and hydration level throughout the experiment.

Both piglets were euthanized at the pre-determined experimental endpoint after 7 days of treatment. On necropsy examination, the only lesions noted in both of these piglets were in the large intestine. Mild hyperemia, dilatation, and mesocolonic edema were present, extending from the cecum to rectum. No pseudomembranes or hemorrhages were present.

The clinical signs of disease and gross lesions observed at necropsy were milder than typical for the inoculating dose (10⁶ spores) of *C. difficile.*

### Secretory immunoglobulin group: (piglets #3 and #4)

One piglet (pig #4) had a decreased appetite starting on the second day after initiation of whey powder administration in the milk, before inoculation occurred. The piglet was inoculated at the same time as the other piglets, but the anorexia worsened, and due to increasing lethargy, weakness, and rapid breathing, the piglet was euthanized 1 day post-inoculation. A necropsy examination of the piglet revealed very mild large intestinal lesions associated with *C. difficile* infection, but no other lesions to explain the piglet's condition.

Piglet #3 had a normal appetite for the duration of the experiment. It developed yellow, pasty diarrhea 2 days after inoculation, which progressed to moderate watery, mucoid, yellow diarrhea by day 4 post-inoculation, and continued to the experimental endpoint. This piglet also developed moderate anal area inflammation, which is typically seen in moderate or severe CDI. The piglet did not develop any systemic signs of illness, and was euthanized at the experimental endpoint after 7 days of treatment. On necropsy examination this piglet had moderate hyperemia, dilatation, and mesocolonic edema extending from the spiral colon to the rectum.

Clinical signs of disease and gross lesions were moderate in this piglet, as expected for the inoculating dose of *C. difficile,* and worse than piglets #1 and #2 in the other treatment group.

### Overall Clinical Impression:

Piglets in the group treated with whey powder containing non-denatured secretory immunoglobulins supplemented with retinylpalmitate developed less severe disease than is typical for the inoculating dose given, and had milder clinical signs and large intestinal lesions than piglet #3 in the group treated with secretory immunoglobulin without additional retinylpalmitate, which group was treated, inoculated, and euthanized at the same timepoints. Retinylpalmitate alone would not have any effect in this model.

## Claims

1. An SIg preparation comprising SIgA and/or SIgM and a Vitamin A supplement, for use in the therapy or prophylaxis of mucosal immunoglobulin deficiency.

2. A preparation for use according to claim 1, wherein the SIg is polyclonal or polyreactive SIgA and/or SIgM, preferably derived from milk of animals selected from the group consisting of human, cow, goat, sheep, buffalo, horse, donkey, pig and camel.

3. A preparation for use according to claim 1 or 2, wherein the Vitamin A is a molecule with Vitamin A activity selected from the group consisting of retinal, retinol, retinoids, retinoic acid, such as all trans-retinoic acid or 13-cis-retinoic acid, retinylpalmitate, 3-dehydroretinol, 3-dehydroretinal, beta-carotene, alpha-carotene, gamma-carotene and beta-cryptoxanthineretinol.

4. A preparation for use according to any of claims 1 to 3, which further comprises at least one further supplement selected from the group consisting of Vitamin B2, Vitamin B6, Vitamin B12, Vitamin C, Vitamin D, Vitamin E, zinc and selenium.

5. A preparation for use according to any of claims 1 to 4, in a formulation for mucosal use, preferably oral, nasal, vaginal, intragastric or rectal use.

6. A preparation for use according to any of claims 1 to 5, wherein a subject is treated, who is at risk of infections, allergies and autoimmune diseases.

7. A preparation for use according to claim 6, wherein the subject is suffering from SIgA and/or SIgM deficiency

8. A preparation for use according to any of claims 1 to 6, wherein the immunoglobulin deficiency is SIgA and/or SIgM deficiency.

9. A preparation for use according to any of claims 6 to 8, wherein the subject is treated with an oral preparation comprising a single-dose of 10 mg to 10 g SIgA and/or SIgM.

10. A preparation for use according to any of claims 1 to 9, as liquid, syrup, lozenge, tablet, chewing gum, spray, powder, instant powder, granules, capsules, cream, gel, drops, suspension, emulsion or food product.

11. A preparation for use according to any of claims 1 to 10, comprising at least 40% Slg of the total immunoglobulin content.

12. A preparation for use according to any of claims 1 to 11, wherein prior to treating a subject, the mucosal immunoglobulin deficiency of the subject is determined in a mucosal sample of said subject in comparison to a reference.

13. A preparation for use according to claim 12, wherein the mucosal sample is derived from body fluids selected from the group consisting of saliva, gastric juice, tears, faeces, urine, sweat, gut lavage, middle ear fluid, bronchial lavage and nasal secretions.

## Patentansprüche

1. Slg-Zubereitung, umfassend SIgA und/oder SIgM und ein Vitamin-A-Ergänzungsmittel, zur Verwendung bei der Therapie oder Vorbeugung gegen Schleimhaut-Immungobulinmangel.

2. Zubereitung zur Verwendung nach Anspruch 1, wobei es sich bei dem Slg um polyklonales oder polyreaktives SIgA und/oder SIgM handelt, das vorzugsweise aus Milch von Tieren stammt, die aus der Gruppe bestehend aus Mensch, Kuh, Ziege, Schaf, Büffel, Pferd, Esel, Schwein und Kamel ausgewählt sind.

3. Zubereitung zur Verwendung nach Anspruch 1 oder 2, wobei es sich bei dem Vitamin A um ein Molekül mit Vitamin-A-Aktivität handelt, das aus der Gruppe bestehend aus Retinal, Retinol, Retinoiden, Retinsäure, wie beispielsweise all-trans-Retinsäure oder 13-cis-Retinsäure, Retinylpalmitat, 3-Dehydroretinol, 3-Dehydroretinal, Betacarotin, Alphacarotin, Gammacarotin und Betacryptoxanthinretinol ausgewählt ist.

4. Zubereitung zur Verwendung nach Anspruch 1 bis 3, die ferner mindestens ein weiteres Ergänzungsmittel aufweist, das aus der Gruppe bestehend aus Vitamin B2, Vitamin B6, Vitamin B12, Vitamin C, Vitamin D, Vitamin E, Zink und Selen ausgewählt ist.

5. Zubereitung zur Verwendung nach einem der Ansprüche 1 bis 4 in einer Formulierung für die Schleimhautverwendung, vorzugsweise für die orale, nasale, vaginale, intragastrische oder rektale Verwendung.

6. Zubereitung zur Verwendung nach einem der Ansprüche 1 bis 5, wobei ein Individuum behandelt wird, für das ein Risiko für Infektionen, Allergien und Autoimmunkrankheiten besteht.

7. Zubereitung zur Verwendung nach Anspruch 6, wobei das Individuum an einem SIgA- und/oder SIgM-Mangel leidet.

8. Zubereitung zur Verwendung nach einem der Ansprüche 1 bis 6, wobei es sich bei dem Immunglobulinmangel um einen SIgA- und/oder SIgM-Mangel handelt.

9. Zubereitung zur Verwendung nach einem der Ansprüche 6 bis 8, wobei das Individuum mit einer oralen Zubereitung behandelt wird, die eine Einzeldosis von 10 mg bis 10 g SIgA und/oder SIgM aufweist.

10. Zubereitung zur Verwendung nach einem der Ansprüche 1 bis 9 als Flüssigkeit, Sirup, Pastille, Tablette, Kaugummi, Spray, Pulver, Instantpulver, Granula, Kapseln, Creme, Gel, Drops, Suspension, Emulsion oder Nahrungsmittelprodukt.

11. Zubereitung zur Verwendung nach einem der Ansprüche 1 bis 10, wobei es sich bei mindestens 40 % des Gesamtimmunglobulingehalts um Slg handelt.

12. Zubereitung zur Verwendung nach einem der Ansprüche 1 bis 11, wobei vor dem Behandeln eines Individuums der Schleimhaut-Immunglobulinmangel des Individuums in einer Schleimhautprobe des Individuums im Vergleich zu einer Referenz bestimmt wird.

13. Zubereitung zur Verwendung nach Anspruch 12, wobei die Schleimhautprobe aus Körperflüssigkeiten stammt, die aus der Gruppe bestehend aus Speichel, Magensaft, Tränenflüssigkeit, Fäzes, Urin, Schweiß, Darmlavage, Mittelohrflüssigkeit, Bronchiallavage und Nasensekret ausgewählt ist.

## Revendications

1. Préparation de SIg comprenant une SIgA et/ou une SIgM et un supplément de vitamine A, pour son utilisation dans la thérapie ou la prophylaxie d'une déficience en immunoglobuline muqueuse.

2. Préparation pour son utilisation selon la revendication 1, dans laquelle la SIg est une SIgA et/ou une SIgM polyclonales ou polyréactives, de préférence dérivées de lait d'animaux sélectionnés dans le groupe constitué des humains, des vaches, des chèvres, des moutons, des buffles, des chevaux, des ânes, des porcs et des chameaux.

3. Préparation pour son utilisation selon la revendication 1 ou 2, dans laquelle la vitamine A est une molécule avec une activité de vitamine A sélectionnée dans le groupe constitué du rétinal, du rétinol, des rétinoïdes, de l'acide rétinoïque, par exemple l'acide tout trans-rétinoïque ou l'acide 13-cis-rétinoïque, du palmitate de rétinyle, du 3-déshydrorétinol, du 3-déshydrorétinal, du bêta-carotène, de l'alpha-carotène, du gamma-carotène et du bêta-cryptoxanthinerétinol.

4. Préparation pour son utilisation selon l'une quelconque des revendications 1 à 3, qui comprend en outre au moins un autre supplément sélectionné dans le groupe constitué de la vitamine B2, de la vitamine B6, de la vitamine B12, de la vitamine C, de la vitamine D, de la vitamine E, du zinc et du sélénium.

5. Préparation pour son utilisation selon l'une quelconque des revendications 1 à 4, dans une formulation pour une utilisation muqueuse, de préférence une utilisation orale, nasale, vaginale, intragastrique ou rectale.

6. Préparation pour son utilisation selon l'une quelconque des revendications 1 à 5, dans laquelle un sujet est traité, qui est à risque d'infections, d'allergies et de maladies auto-immunes.

7. Préparation pour son utilisation selon la revendication 6, dans laquelle le sujet souffre d'une déficience en SIgA et/ou en SIgM.

8. Préparation pour son utilisation selon l'une quelconque des revendications 1 à 6, dans laquelle la déficience en immunoglobulines est une déficience en SIgA et/ou en SIgM.

9. Préparation pour son utilisation selon l'une quelconque des revendications 6 à 8, dans laquelle le sujet est traité avec une préparation orale comprenant une dose unique de 10 mg à 10 g de SIgA et/ou SIgM.

10. Préparation pour son utilisation selon l'une quelconque des revendications 1 à 9, sous la forme d'un liquide, d'un sirop, d'une pastille, d'un comprimé, d'un chewinggum, d'une pulvérisation, d'une poudre, d'une poudre instantanée, de granules, de capsules, d'une crème, d'un gel, de gouttes, d'une suspension, d'une émulsion ou d'un produit alimentaire.

11. Préparation pour son utilisation selon l'une quelconque des revendications 1 à 10, comprenant au moins 40 % de SIg de la teneur totale en immunoglobuline.

12. Préparation pour son utilisation selon l'une quelconque des revendications 1 à 11, dans laquelle avant le traitement d'un sujet, la déficience en immunoglobuline muqueuse du sujet est déterminée dans un échantillon de muqueuse prélevé sur ledit sujet par comparaison à une référence.

13. Préparation pour son utilisation selon la revendication 12, dans laquelle l'échantillon de muqueuse est dérivé de fluides corporels sélectionnés dans le groupe constitué de la salive, du suc gastrique, des larmes, des selles, de l'urine, de la transpiration, d'un lavage gastrique, du fluide de l'oreille moyenne, d'un lavage bronchique et des sécrétions nasales.


### SEQUENCE LISTING

<110> Himmler, Gottfried
<120> SECRETORY IMMUNOGLOBULIN DEFICIENCY TREATMENT AND PROPHYLAXIS
<130> HI002P
<160> 6
<170> PatentIn version 3.5
<210> 1
   <211> 4
   <212> PRT
   <213> Artificial Sequence
<220>
   <223> Peptid
<220>
   <221> VARIANT
   <222> (2)..(2)
   <223> Xaa can be any naturally occurring amino acid but proline
<220>
   <221> VARIANT
   <222> (4)..(4)
   <223> Xaa can be any naturally occurring amino acid but proline
<400> 1
   Asn Xaa Thr Xaa
   1
<210> 2
   <211> 4
   <212> PRT
   <213> Artificial Sequence
<220>
   <223> Peptid
<220>
   <221> VARIANT
   <222> (2)..(2)
   <223> Xaa can be any naturally occurring amino acid but proline
<220>
   <221> VARIANT
   <222> (4)..(4)
   <223> Xaa can be any naturally occurring amino acid but proline
<400> 2
   Asn Xaa Ser Xaa
   1
<210> 3
   <211> 471
   <212> PRT
   <213> Artificial Sequence
<220>
   <223> heavy chain antibody sequence
<400> 3
   Glu Val Gln Leu Gln Gln Ser Gly Pro Glu Leu Val Lys Pro Gly Ala
   1               5                   10                  15
   Ser Met Lys Ile Ser Cys Lys Ala Ser Gly Tyr Ser Phe Thr Gly Tyr
               20                  25                  30
   Thr Met Asn Trp Val Lys Gln Ser His Gly Lys Asn Leu Glu Trp Ile
           35                  40                  45
   Gly Leu Ile Asn Pro Tyr Asn Gly Gly Thr Ser Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Asp Val Arg Gly Ala Trp Phe Ala Tyr Trp Gly Gln Gly Thr
               100                 105                 110
   Leu Val Thr Val Ser Ala Ala Ser Pro Thr Ser Pro Lys Val Phe Pro
           115                 120                 125
   Leu Ser Leu Cys Ser Thr Gln Pro Asp Gly Asn Val Val Ile Ala Cys
       130                 135                 140
   Leu Val Gln Gly Phe Phe Pro Gln Glu Pro Leu Ser Val Thr Trp Ser
   145                 150                 155                 160
   Glu Ser Gly Gln Gly Val Thr Ala Arg Asn Phe Pro Pro Ser Gln Asp
                   165                 170                 175
   Ala Ser Gly Asp Leu Tyr Thr Thr Ser Ser Gln Leu Thr Leu Pro Ala
               180                 185                 190
   Thr Gln Cys Leu Ala Gly Lys Ser Val Thr Cys His Val Lys His Tyr
           195                 200                 205
   Thr Asn Pro Ser Gln Asp Val Thr Val Pro Cys Pro Val Pro Ser Thr
       210                 215                 220
   Pro Pro Thr Pro Ser Pro Ser Thr Pro Pro Thr Pro Ser Pro Ser Cys
   225                 230                 235                 240
   Cys His Pro Arg Leu Ser Leu His Arg Pro Ala Leu Glu Asp Leu Leu
                   245                 250                 255
   Leu Gly Ser Glu Ala Asn Leu Thr Cys Thr Leu Thr Gly Leu Arg Asp
               260                 265                 270
   Ala Ser Gly Val Thr Phe Thr Trp Thr Pro Ser Ser Gly Lys Ser Ala
           275                 280                 285
   Val Gln Gly Pro Pro Glu Arg Asp Leu Cys Gly Cys Tyr Ser Val Ser
       290                 295                 300
   Ser Val Leu Pro Gly Cys Ala Glu Pro Trp Asn His Gly Lys Thr Phe
   305                 310                 315                 320
   Thr Cys Thr Ala Ala Tyr Pro Glu Ser Lys Thr Pro Leu Thr Ala Thr
                   325                 330                 335
   Leu Ser Lys Ser Gly Asn Thr Phe Arg Pro Glu Val His Leu Leu Pro
               340                 345                 350
   Pro Pro Ser Glu Glu Leu Ala Leu Asn Glu Leu Val Thr Leu Thr Cys
           355                 360                 365
   Leu Ala Arg Gly Phe Ser Pro Lys Asp Val Leu Val Arg Trp Leu Gln
       370                 375                 380
   Gly Ser Gln Glu Leu Pro Arg Glu Lys Tyr Leu Thr Trp Ala Ser Arg
   385                 390                 395                 400
   Gln Glu Pro Ser Gln Gly Thr Thr Thr Phe Ala Val Thr Ser Ile Leu
                   405                 410                 415
   Arg Val Ala Ala Glu Asp Trp Lys Lys Gly Asp Thr Phe Ser Cys Met
               420                 425                 430
   Val Gly His Glu Ala Leu Pro Leu Ala Phe Thr Gln Lys Thr Ile Asp
           435                 440                 445
   Arg Leu Ala Gly Lys Pro Thr His Val Asn Val Ser Val Val Met Ala
       450                 455                 460
   Glu Val Asp Gly Thr Cys Tyr
   465 470
<210> 4
   <211> 458
   <212> PRT
   <213> Artificial Sequence
<220>
   <223> heavy chain antibody sequence
<400> 4
   Glu Val Gln Leu Gln Gln Ser Gly Pro Glu Leu Val Lys Pro Gly Ala
   1               5                   10                  15
   Ser Met Lys Ile Ser Cys Lys Ala Ser Gly Tyr Ser Phe Thr Gly Tyr
               20                  25                  30
   Thr Met Asn Trp Val Lys Gln Ser His Gly Lys Asn Leu Glu Trp Ile
           35                  40                  45
   Gly Leu Ile Asn Pro Tyr Asn Gly Gly Thr Ser Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Asp Val Arg Gly Ala Trp Phe Ala Tyr Trp Gly Gln Gly Thr
               100                 105                 110
   Leu Val Thr Val Ser Ala Ala Ser Pro Thr Ser Pro Lys Val Phe Pro
           115                 120                 125
   Leu Ser Leu Asp Ser Thr Pro Gln Asp Gly Asn Val Val Val Ala Cys
       130                 135                 140
   Leu Val Gln Gly Phe Phe Pro Gln Glu Pro Leu Ser Val Thr Trp Ser
   145                 150                 155                 160
   Glu Ser Gly Gln Asn Val Thr Ala Arg Asn Phe Pro Pro Ser Gln Asp
                   165                 170                 175
   Ala Ser Gly Asp Leu Tyr Thr Thr Ser Ser Gln Leu Thr Leu Pro Ala
               180                 185                 190
   Thr Gln Cys Pro Asp Gly Lys Ser Val Thr Cys His Val Lys His Tyr
           195                 200                 205
   Thr Asn Pro Ser Gln Asp Val Thr Val Pro Cys Pro Val Pro Pro Pro
       210                 215                 220
   Pro Pro Cys Cys His Pro Arg Leu Ser Leu His Arg Pro Ala Leu Glu
   225                 230                 235                 240
   Asp Leu Leu Leu Gly Ser Glu Ala Asn Leu Thr Cys Thr Leu Thr Gly
                   245                 250                 255
   Leu Arg Asp Ala Ser Gly Ala Thr Phe Thr Trp Thr Pro Ser Ser Gly
               260                 265                 270
   Lys Ser Ala Val Gln Gly Pro Pro Glu Arg Asp Leu Cys Gly Cys Tyr
           275                 280                 285
   Ser Val Ser Ser Val Leu Pro Gly Cys Ala Gln Pro Trp Asn His Gly
       290                 295                 300
   Glu Thr Phe Thr Cys Thr Ala Ala His Pro Glu Leu Lys Thr Pro Leu
   305                 310                 315                 320
   Thr Ala Asn Ile Thr Lys Ser Gly Asn Thr Phe Arg Pro Glu Val His
                   325                 330                 335
   Leu Leu Pro Pro Pro Ser Glu Glu Leu Ala Leu Asn Glu Leu Val Thr
               340                 345                 350
   Leu Thr Cys Leu Ala Arg Gly Phe Ser Pro Lys Asp Val Leu Val Arg
           355                 360                 365
   Trp Leu Gln Gly Ser Gln Glu Leu Pro Arg Glu Lys Tyr Leu Thr Trp
       370                 375                 380
   Ala Ser Arg Gln Glu Pro Ser Gln Gly Thr Thr Thr Phe Ala Val Thr
   385                 390                 395                 400
   Ser Ile Leu Arg Val Ala Ala Glu Asp Trp Lys Lys Gly Asp Thr Phe
                   405                 410                 415
   Ser Cys Met Val Gly His Glu Ala Leu Pro Leu Ala Phe Thr Gln Lys
               420                 425                 430
   Thr Ile Asp Arg Met Ala Gly Lys Pro Thr His Val Asn Val Ser Val
           435                 440                 445
   Val Met Ala Glu Val Asp Gly Thr Cys Tyr
   450 455
<210> 5
   <211> 453
   <212> PRT
   <213> Artificial Sequence
<220>
   <223> heavy chain antibody sequence
<400> 5
   Glu Val Gln Leu Gln Gln Ser Gly Pro Glu Leu Val Lys Pro Gly Ala
   1               5                   10                  15
   Ser Met Lys Ile Ser Cys Lys Ala Ser Gly Tyr Ser Phe Thr Gly Tyr
               20                  25                  30
   Thr Met Asn Trp Val Lys Gln Ser His Gly Lys Asn Leu Glu Trp Ile
           35                  40                  45
   Gly Leu Ile Asn Pro Tyr Asn Gly Gly Thr Ser Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Leu Ser Leu Thr Ser Glu Asp Ser Ala Val Tyr Tyr Cys
                   85                  90                  95
   Ala Arg Asp Val Arg Gly Ala Trp Phe Ala Tyr Trp Gly Gln Gly Thr
               100                 105                 110
   Leu Val Thr Val Ser Ala Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Asp Glu Tyr Leu His Gly Asp Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Ala Arg Tyr
                   405                 410                 415
   Ser Pro Arg Met Leu Arg Trp Ala His Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
   450
<210> 6
   <211> 216
   <212> PRT
   <213> Artificial Sequence
<220>
   <223> light chain antibody sequence
<400> 6
   Gln Ala Val Val Thr Gln Glu Ser Ala Leu Thr Thr Ser Pro Gly Glu
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Asp His Leu Phe Thr Gly
           35                  40                  45
   Leu Ile Gly Gly Thr Asn Asn Arg Ala Pro Gly Val Pro Ala Arg Phe
       50                  55                  60
   Ser Gly Ser Leu Ile Gly Asp Lys Ala Ala Leu Thr Ile Thr Gly Ala
   65                  70                  75                  80
   Gln Thr Glu Asp Glu Ala Ile Tyr Phe Cys Ala Leu Trp Tyr Ser Asn
                   85                  90                  95
   His Arg Val Phe Gly Gly Gly Thr Lys Leu Thr Val Leu Arg Thr Val
               100                 105                 110
   Ala Ala Pro Ser Val Phe Ile Phe Pro Pro Ser Asp Glu Gln Leu Lys
           115                 120                 125
   Ser Gly Thr Ala Ser Val Val Cys Leu Leu Asn Asn Phe Tyr Pro Arg
       130                 135                 140
   Glu Ala Lys Val Gln Trp Lys Val Asp Asn Ala Leu Gln Ser Gly Asn
   145                 150                 155                 160
   Ser Gln Glu Ser Val Thr Glu Gln Asp Ser Lys Asp Ser Thr Tyr Ser
                   165                 170                 175
   Leu Ser Ser Thr Leu Thr Leu Ser Lys Ala Asp Tyr Glu Lys His Lys
               180                 185                 190
   Val Tyr Ala Cys Glu Val Thr His Gln Gly Leu Ser Ser Pro Val Thr
           195                 200                 205
   Lys Ser Phe Asn Arg Gly Glu Cys
       210                 215